# EUROPEAN PATENT SPECIFICATION

(11) **EP 0 359 418 B1**
(45) Date of publication and mention of the grant of the patent: **12.10.1994**
(21) Application number: 89308506.8
(22) Date of filing: 22.08.1989
(51) Int. Cl.: C07D 413/04, A61K 31/42, C07D 263/20, C07D 413/14

(54) **5'-Indolinyl-5beta-amidomethyloxazolidin-2-ones, 3-(fused-ring substituted)phenyl-5beta-amidomethyloxazolidin-2-ones and 3-(nitrogen substituted)phenyl-5beta-amidomethyloxazolidin-2-ones**
5-Indolinyl-5-beta-amidomethyloxazolidin-2-one, 3-(substituierte kondensierte)phenyl-5-beta amidomethyloxazolidin-2-one und 3-(Nitrogen-substituierte)phenyl-5-beta-amidomethyloxazolidin-2-one
5-Indolinyl-5-bêta amidométhyloxazolidines-2-one, 3-(phényl condensé)-5 bêta amidométhyloxazolidines-2-ones et 3-phényl-azoté-5 bêta amidométhyloxazolidine 2-one

(30) Priority: 15.09.1988 US 244988; 05.10.1988 US 253850; 17.03.1989 US 324942
(43) Date of publication of application: 21.03.1990
(60) Divisional application: 94102762.5
(73) Proprietor: THE UPJOHN COMPANY, Kalamazoo, Michigan 49001 (US)
(72) Inventor: Brickner, Steven L., Portage Michigan 49081 (US)
(74) Representative: Perry, Robert Edward

(56) References cited:
- EP-A- 0 127 902
- EP-A- 0 184 170
- EP-A- 0 311 090

## Description

### Field of the Invention

The present invention relates to 5'-indolinyloxazolidinones which are useful as antibacterial agents.

### Background of the Invention

US-A-4128654 discloses 5-halomethylphenyl-2-oxazolidinones which are useful in controlling fungal and bacterial diseases of plants.

US-A-4250318 discloses 3-substituted phenyl-5-hydroxymethyloxazolidinones having antidepressant utility.

US-B-0029607 discloses 3-substituted phenyl-5-hydroxymethyloxazolidinones having antidepressant, tranquillising and sedative utility.

US-A-4340606 discloses 3-(p-alkylsulfonyl)phenyl-5-(hydroxymethyl or acyloxymethyl)oxazolidinones having antibacterial activity in mammals.

BE-A-0892270 discloses 3-(arylalkyl, arylalkenyl or arylacetylenic-substituted phenyl)-5-(aminomethyl)oxazolidinones which are inhibitors of monoamine oxidase.

US-A-4461773 discloses 3-substituted phenyl-5-hydroxymethyloxazolidinones which have antibacterial activity.

EP-A-0127902 and EP-A-0184170 disclose 3-substituted phenyl-5-amidomethyloxazolidinones which have antibacterial utility.

EP-A-0311090 (= US-A-4705799) discloses aminomethyloxo-oxazolidinyl benzene derivatives including sulfides, sulfoxides, sulfones and sulfonamides which possess antibacterial activity.

US-A-4801600 discloses 6'-indolinyloxazolidinones (where the indolinyl nitrogen is meta to the oxazolidinone nitrogen), both generically, see formula (I) where "X" is NR₆, and specifically, see Example 13.

### SUMMARY OF THE INVENTION

Disclosed are 5'-indolinyloxazolidin-2-ones of formula (XI), i.e. where the indolinyl nitrogen is para to the oxazolidinone nitrogen. In formule XI:
(I) R₁ is -H,
   C₁-C₁₂ alkyl optionally substituted with 1-3 Cl,
   C₃-C₁₂ cycloalkyl,
   C₅-C₁₂ alkenyl containing one double bond,
   - φ optionally substituted with 1-3 -OH, -OCH₃, -OC₂H₅, -NO₂, -F, -Cl, -Br, -COOH and -SO₃H, -N(R₁₋₄)(R₁₋₅) where R₁₋₄ and R₁₋₅ are the same or different and are -H, C₁-C₂ alkyl,
   furanyl,
   tetrahydrofuranyl,
   2-thiophene,
   pyrrolidinyl,
   pyridinyl,
   -O-R₁₋₆ where R₁₋₆ is C₁-C₄ alkyl,
   -NH₂,
   -NHR₁₋₆ where R₁₋₆ is as defined above,
   -NR₁₋₆R₁₋₇ where R₁₋₇ is C₁-C₃ alkyl and R₁₋₆ is as defined above, and where R₁₋₆ and R₁₋₇ can be taken together with the attached nitrogen atom to form a saturated mono-nitrogen C₅-C₇ heterocyclic ring including -O- (morpholine),
   -CH₂-OH,
   -CH₂-OR₁₋₈ where R₁₋₈ is C₁-C₄ alkyl or -CO-R₁₋₉ where R₁₋₉ is C₁-C₄ alkyl or -φ;
(II) two of R₂, R₃ and R₄ are -H and the other of R₂, R₃ and R₄ is -H,
   -F, -Cl, Br, -I,
   C₁-C₆ alkyl,
   -OH,
   -CO-O-R₂₋₁ where R₂₋₁ is C₁-C₄ alkyl or -φ,
   -O-R₂₋₁ where R₂₋₁ is as defined above,
   -COOH,
   -COO⁻,
   -CHO,
   -CO-NR₂₋₂R₂₋₃ where R₂₋₂ and R₂₋₃ are the same or different and are -H, C₁-C₃ alkyl, or R₂₋₂ and R₂₋₃ are taken together with the attached nitrogen atom to form a saturated mono-nitrogen C₃-C₆ heterocyclic ring optionally containing -O-,
   -C≡N,
   -C≡CH,
   -N≡C,
   -CHOH-CH₃,
   -CO-CH₃,
   -SH,
   -SO-CH₃,
   -SO-φ,
   -S-CH₃,
   -S-φ,
   -SO₂-CH₃,
   -SO₂-φ,
   -SO₃H,
   -SO₂-NH₂,
   -N₃,
   -NO₂
   -NR_{2-4R2-5} where R₂₋₄ and R₂₋₅ are the same or different and are -H and C₁-C₃ alkyl,
   -NH-CO-R₂₋₆ where R₂₋₆ is C₁-C₄ alkyl or -φ,
   -NH-CO-NH₂,
   -CH=CH₂,
   -CH₂-CH=CH₂,
   -CH=N-OH,
   -CH=N-OCH₃, 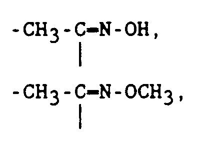 -C*H-CH₂-O* where the atoms marked with the asterisk (*) are bonded to each other resulting in the formation of a ring,
   where
(III) R₅ is -H,
   C₁-C₁₂ alkyl,
   -CH₂-φ,
   -CH₂CH₂-φ,
   C₃-C₇ cycloalkyl,
   C₂-C₁₂ alkenyl containing from 1 thru 3 double bonds,
   C₂-C₁₂ alkynyl containing 1 triple bond,
   -CHO,
   -CO-R₅₋₁ where R₅₋₁ is
   (A) C₁-C₆ alkyl optionally substituted with 1 -O-CH₃, -φ, -COOH, -NH₂, -SO₃H or 1-3 -Cl,
   (B) C₃-C₇ cycloalkyl,
   (C) 2-pyridinyl, 2-thiophene, 2-thiophenemethyl or 2-pyrrole,
   (D) -φ optionally substituted with 1-3 -F, -Cl, Br, -I,
      C₁-C₆ alkyl,
      -OH,
      -CO-O-R₅₋₂ where R₅₋₂ is C₁-C₄ alkyl or -φ,
      -O-R₅₋₂ where R₅₋₂ is as defined above,
      -COOH,
      -CO-NR₅₋₃R₅₋₄ where R₅₋₃ and R₅₋₄ are the same or different and are -H, C₁-C₃ alkyl, or R₅₋₃ and R₅₋₄ are taken together with the attached nitrogen atom to form a saturated mononitrogen C₃-C₆ heterocyclic ring optionally containing -O-,
      -C≡N, -CHOH-CH₃,
      -CO-CH₃,
      -SH,
      -SO-CH₃,
      -SO-φ,
      -S-CH₃,
      -S-φ,
      -SO₂-CH₃,
      -SO₂-φ,
      -SO₃H,
      -SO₂-NH₂,
      -N₃,
      -NO₂,
      -NR_{5-5R5-6} where R₅₋₅ and R₅₋₆ are the same or different and are -H and C₁-C₃ alkyl,
      -NH-CO-R₅₋₇ where R₅₋₇ is C₁-C₄ alkyl or -φ,
   -CO-O-R₅₋₈ where R₅₋₈ is C₁-C₄ alkyl or -φ either optionally substituted with 1 or 2
   -F, -Cl, Br, -I,
   C₁-C₆ alkyl,
   -OH,
   -CO-O-R₅₋₂ where R₅₋₂ is as defined above,
   -O-R₅₋₂ where R₅₋₂ is as defined above,
   -COOH,
   -CO-NR₅₋₃R₅₋₄ where R₅₋₃ and R₅₋₄ are as defined above,
   -C≡N,
   -CHOH-CH₃,
   -CO-CH₃,
   -SH,
   -SO-CH₃,
   -SO-φ,
   -S-CH₃,
   -S-φ,
   -SO₂-CH₃,
   -SO₂-φ,
   -SO₃H,
   -SO₂-NH₂,
   -N₃,
   -NO₂,
   -NR_{5-5R5-6} where R₅₋₅ and R₅₋₆ are as defined above,
   -NH-CO-R₅₋₇ where R₅₋₇ is as defined above,
   -CO-N(R₅₋₉)₂ where R₅₋₉ is -H or R₅₋₈ as defined above and where the R₅₋₉'s can be taken together with the attached nitrogen atom to form a saturated mononitrogen C₃-C₆ heterocyclic ring optionally containing -O- or -NH-,
   -CO-CH₂-CN,
   -CO-CH₂-OH,
   -CO-CH₂-O-φ where φ is optionally substituted with 1-3 -O-CH₃, 1 -NO₂ and 1 -NH₂,
   -CO-CH₂-O-R₅₋₁₀ where R₅₋₁₀ is
   C₁-C₆ alkyl optionally substituted with -φ,
   -φ optionally substituted with 1-3 -O-CH₃, 1 -NO₂ and -NH₂,
   -CO-R₅₋₁₁ where R₅₋₁₁ is C₁-C₆ alkyl, -φ optionally substituted with 1-4 -F, 1-3 -Cl, 1 -OCH₃, -OH, -NH₂, -NO₂, -CO-CH₃, -SO₂-CH₃ and -SO₂-OH,
   -CO-CH(NH-CO-R₅₋₁₂)-R₅₋₁₃ where R₅₋₁₃ is -H or -CH₃ and R₅₋₁₂ is C₁-C₆ alkyl, -φ optionally substituted with 1 or 2 -OH, -OCH₃, -NO₂, -NH₂, -Cl, -F, -NH-CH₃ and -N(CH₃)₂,
   -CO-CHNH₂-R₅₋₁₄ where R₅₋₁₄ is -CH(CH₃)₂, -CH₂-CH(CH₃)₂, -CHCH₃-CH₂-CH₃, -CH₂-OH, -CH(OH)-CH₃, -CH₂-φ, -CH₂-φ-OH, -CH₂-SH, -CH₂-CH₂-S-CH₃, and -CH₂-COOH,
   -SO₂-CH₃,
   -SO₂-φ,
   -SO₃H,
   and R₆ is -H and C₁-C₃ alkyl and pharmaceutically acceptable salts thereof.

Further disclosed are compounds selected from the group consisting of a 5′-indolinyloxazolidin-2-ones of formula (XIII) where
X₁ is -CO-CH₃, -CO-O-C(CH₃)₃, -CO-O-CH₂-φ and -CO-O-(CH₂)₂-Si(CH₃)₃,
R₆ is -I, -N₃ and -NH₂ and where R₂, R₃ and R₄ are as defined above, and salts thereof.

Further intermediates for the compounds of the invention have formulae VI, VII and VIII (see Chart A), wherein R₂, R₃ and R₄ are as defined above and X₁ is -CO-CH₃, -CO-O-C(CH₃)₃, -CO-O-CH₂-φ or -CO-O-(CH₂)₂-Si(CH₃)₃; or a salt thereof.

Throughout the specification, φ is used to represent phenyl.

### DETAILED DESCRIPTION OF THE INVENTION

The 5'-indolinyloxazolidin-2-ones (XI) are prepared starting with the corresponding 5-nitroindolines (I). It is preferred that R₂, R₃ and R₄ all be -H. The indolinyl nitrogen of the 5-nitroindolines (I) is protected to produce the corresponding protected 5-nitroindolines (II). Suitable protecting agents, X₁, include t-butyloxycarbonyl (BOC), acetyl, -CO-O-CH₂-φ and -CO-O-(CH₂)₂-Si(CH₃)₃. It is preferred that X₁ be t-butyloxycarbonyl. Next, the nitro group of the protected 5-nitroindolines (II) is reduced with hydrogen and an appropriate catalyst such as palladium on carbon to the corresponding protected 5-aminoindolines (III). Acylation of the free unprotected 5-amino group of the 1-protected 5-aminoindolines (III) with a carbobenzyloxy (CBZ) group gives the urethanes (IV). The urethanes (IV) are then reacted with Br-CH₂-CH=CH₂ in THF and a base forming the N-allyl-N-CBZ compounds (V). Suitable bases include sodium hydride, sodium methoxide, potassium tertiary butoxide and lithium diisopropylamide; preferred is sodium hydride. The N-allyl-N-CBZ compounds (V) are cyclized to form the oxazolidinone nucleus by reaction with an electrophilic agent. Suitable electrophilic agents include bromine and iodine; iodine in chloroform is preferred. The oxazolidinone nucleus formed is the protected 5-iodomethyloxazolidin-2-one (VI). Following formation of the oxazolidinone ring, the desired side chain at the 5-position is formed by reacting the protected 5-iodomethyl oxazolidinones (VI) with an azide to form the protected asides (VII). The protected asides (VII) are reduced with hydrogen in the presence of a catalyst such as palladium or by Pφ₃ or H₂S or other methods known to those skilled in the art to give racemic protected 5-aminomethyloxazolidin-2-ones (VIII). The racemic compounds can be resolved at the aminomethyloxazolidinone (VIII) stage using methods known to those skilled in the art, see for example, Optical Resolution Procedures for Chemical Compounds, Vol 1,: Amines and Related Compounds, Paul Newman, Optical Resolution Information Center, Manhattan College, Riverdale, NY, 10471, 1978. For example, treatment of the d,1-aminomethyloxazolidinone (VIII) mixture with an optically active acid such as (+)-tartaric acid or alternatively with (-)-tartaric acid, would yield a mixture of diastereomeric salts, which can be separated most conviently by fractional crystallization to give a salt containing only one enantiomer of the racemic mixture. Other suitable optically active acids include, (-) dibenzoyltartaric acid, (+)-camphoric acid, (+)-and (-)-malic acid and (+)-camphor-10-sulfonic acid. By reacting the diastereomeric salt with a base one obtains the enantiomer as the free compound. These compounds are then acylated to produce the protected 5'-indolinyloxazolidin-2-ones (IX) containing the desired R₁ group. It is preferred that R₁ is H, C₁-C₆ alkyl, C₃-C₆ cycloalkyl, -OCH₃ and -CHCl₂; it is more preferred that R₁ is -CH₃. Acid hydrolysis of the (BOC) protected 5'-indolinyloxazolidi-2-nones (IX) produces the unprotected 5'-indolinyloxazolidin-2-ones (X) which are then N-acylated or N-alkylated, if necessary, with the desired R₅ group, either as the acid halide, anhydride, or through a reductive alkylation sequence to produce the desired 5'-indolinyloxazolidin-2-ones (XI). The CBZ protecting group is removed by hydrogen with palladium on carbon and the -CO-O-(CH₂)₂-Si(CH₃)₃ is removed by tetra-butylammonium fluoride, see J. Chem. Soc. Chem. Commun., 358 (1970). For the 5'-indolinyloxazolidin-2-ones (XI), it is preferred that R₅ is -CH₃, -CH₂-CH=CH₂, -CH₂-C≡CH, -CHO, -CO-R₅₋₁ where R₅₋₁ is -CH₃, -C₂H₅, -CH(CH₃)₂, -CH₂Cl, -CHCl₂, -CH₂-OH, -CH₂-O-CH₃, 2-thienyl and cyclopropyl. It is more preferred that R₅ is -CH₃, -CH₂-CH=CH₂, -CHO, -CO-R₅₋₁ where R₅₋₁ is -CH₃, -C₂H₅, -CHCl₂, -CH₂-OH and 2-thienyl.

The 5'-indolinyloxazolidin-2-ones (XI) have an asymmetric centre at the C₅-position of the oxazolidinone ring which produces two enantiomers. The mixture of enantiomers is resolved by means known to those skilled in the art. The enantiomer which is pharmacologically active is the β-enantiomer; see Chart A. The racemic mixture is useful in the same way and for the same purpose as the pure β-enantiomer; the difference is that twice as much racemic material must be used to produce the same effect as the pure β-enantiomer.

The 5'-indolinyloxazolidin-2-ones of the present invention are useful as antibacterial agents in treating infections in mammals caused by gram-positive and anaerobic infections. It is preferred to treat humans and useful warm-blooded mammals such as cattle, horses, sheep, hogs, dogs, cats, etc.

The 5'-indolinyloxazolidin-2-ones (XI) of the present invention are also useful in treating AIDS patients infected with *Mycobacterium avium*.

The 5'-indolinyloxazolidin-2-ones (XI) can be administered either parenterally (IV, IM, SQ) or orally. The daily dose is about 5 to about 20 mg/kg. This dose can preferrably be given in divided doses and administered 2-4 times daily. The preferred route of administration as well as the particular dosage form for either the parenteral or oral route depends on the particular facts of the situation including the nature of the infection and condition of the patient. The usual pharmaceutical dosage forms appropriate for parenteral (solution, suspension in oil) and oral (tablet, capsule, syrup, suspension, etc) administration are known to those skilled in the art and there is nothing unusual about using those dosage forms with the 5'-indolinyloxazolidin-2-ones (XI). The exact dosage of the 5'-indolinyloxazolidin-2-ones (XI) to be administered, the frequency of administration, route of administration, and the dosage form will vary depending on a number of factors known to those skilled in the art including the age, weight, sex, general physical condition of the patient, the nature of the infection (particular microorganism involved, its virulence, the extent of the infection) other medical problems of the patient, etc as is well known to the physican treating infectious diseases.

The 5'-indolinyloxazolidin-2-ones (XI) can be used either alone or in conjunction with other antibacterial agents as is known to those skilled in the art. Further, the 5'-indolinyloxazolidin-2-ones (XI) can be used in conjunction with non-antibacterial agents as is known to those skilled in the art.

Suitable pharmaceutically acceptable salts include, for example, chloride, sulfate, phosphate, citrate and oxylate.

### DEFINITIONS AND CONVENTIONS

The definitions and explanations below are for the terms as used throughout this entire document including both the specification and the claims.

### I. CONVENTIONS FOR FORMULAS AND DEFINITIONS OF VARIABLES

The chemical formulas representing various compounds or molecular fragments in the specification and claims may contain variable substituents in addition to expressly defined structural features. These variable substituents are identified by a letter or a letter followed by a numerical subscript, for example, "Z₁" or "Rᵢ" where "i" is an integer. These variable substituents are either monovalent or bivalent, that is, they represent a group attached to the formula by one or two chemical bonds. For example, a group Z₁ would represent a bivalent variable if attached to the formula CH₃-C(=Z₁)H. Groups Rᵢ and Rⱼ would represent monovalent variable substituents if attached to the formula CH₃-CH₂-C(Rᵢ)(Rⱼ)H₂. When chemical formulas are drawn in a linear fashion, such as those above, variable substituents contained in parentheses are bonded to the atom immediately to the left of the variable substituent enclosed in parenthesis. When two or more consecutive variable substituents are enclosed in parenthesis, each of the consecutive variable substituents is bonded to the immediately preceding atom to the left which is not enclosed in parentheses. Thus, in the formula above, both Rᵢ and Rⱼ are bonded to the preceding carbon atom.

Chemical formulas or portions thereof drawn in a linear fashion represent atoms in a linear chain. The symbol "-" in general represents a bond between two atoms in the chain. Thus CH₃-O-CH₂-CH(Rᵢ)-CH₃ represents a 2-substituted-1-methoxypropane compound. In a similar fashion, the symbol "=" represents a double bond, e.g., CH₂=C(Rᵢ)-O-CH₃, and the symbol "≡" represents a triple bond, e.g., HC≡C-CH(Rᵢ)-CH₂-CH₃. Carbonyl groups are represented in either one of two ways: -CO- or -C(=O)-, with the former being preferred for simplicity.

Chemical formulas or cyclic (ring) compounds or molecular fragments can be represented in a linear fashion. Thus, the compound 4-chloro-2-methylpyridine can be represented in linear fashion by N*=C(CH₃)-CH=CCl-CH=C*H with the convention that the atoms marked with an asterisk (*) are bonded to each other resulting in the formation of a ring. Likewise, the cyclic molecular fragment, 4-(ethyl)-1-piperazinyl can be represented by -N*-(CH₂)₂-N(C₂H₅)-CH₂-C*H₂.

A rigid cyclic (ring) structure for any compounds herein defines an orientation with respect to the plane of the ring for substituents attached to each carbon atom of the rigid cyclic compound. For saturated compounds which have two substituents attached to a carbon atom which is part of a cyclic system, -C(X₁)(X₂)- the two substituents may be in either an axial or equatorial position relative to the ring and may change between axial/equatorial. However, the position of the two substituents relative to the ring and each other remains fixed. While either substituent at times may lie in the plane of the ring (equatorial) rather than above or below the plane (axial), one substituent is always above the other. In formulas depicting such compounds, a substituent (X₁) which is "below" another substituent (X₂) will be identified as being in the alpha (α) configuration and is identified by a broken, dashed or dotted line attachment to the carbon atom, i.e., by the symbol "- - -" or "...". The corresponding substituent attached "above" (X₂) and other (X₁) is identified as being in the beta (β) configuration and is indicated by an unbroken line attachment to the carbon atom.

When a variable substituent is bivalent, the valences may be taken together or separately or both in the definition of the variable. For example, a variable Rᵢ attached to a carbon atom as -C(=Rᵢ)- might be bivalent and be defined as oxo or keto (thus forming a carbonyl group (-CO-) or as two separately attached monovalent variable substituents α-Rᵢ₋ⱼ and β-Rᵢ₋ₖ. When a bivalent variable, Rᵢ, is defined to consist of two monovalent variable substituents, the convention used to define the bivalent variable is of the form "α-Rᵢ₋ⱼ:β-Rᵢ₋ₖ" or some variant thereof. In such a case both α-Rᵢ₋ⱼ and β-Rᵢ₋ₖ are attached to the carbon atom to give -C(α-Rᵢ₋ⱼ)(β-Rᵢ₋ₖ)-. For example, when the bivalent variable R₆, -C(=R₆)- is defined to consist of two monovalent variable substituents, two monovalent variable substituents are α-R₆₋₁:β-R₆₋₂, .... α-R₆₋₉:β-R₆₋₁₀, etc, giving -C(α-R₆₋₁)(β-R₆₋₂)-, .... -C(α-R₆₋₉) (β-R₆₋₁₀)-, etc. Likewise, for the bivalent variable R₁₁, -C(=R₁₁)-, two monovalent variable substituents are α-R₁₁₋₁:β-R₁₁₋₂. For a ring substituent for which separate α and β orientations do not exist (e.g. due to the presence of a carbon carbon double bond in the ring), and for a substituent bonded to a carbon atom which is not part of a ring the above convention is still used, but the α and β designations are omitted.

Just as a bivalent variable may be defined as two separate monovalent variable substituents, two separate monovalent variable substituents may be defined to be taken together to form a bivalent variable. For example, in the formula -C₁(Rᵢ)H-C₂(Rⱼ)H- (C₁ and C₂ define arbitrarily a first and second carbon atom, respectively) Rᵢ and Rⱼ may be defined to be taken together to form (1) a second bond between C₁ and C₂ or (2) a bivalent group such as oxa (-O-) and the formula thereby describes an epoxide. When Rᵢ and Rⱼ are taken together to form a more complex entity, such as the group -X-Y-, then the orientation of the entity is such that C₁ in the above formula is bonded to X and C₂ is bonded to Y. Thus, by convention the designation "... Rᵢ and Rⱼ are taken together to form -CH₂-CH₂-O-CO- ..." means a lactone in which the carbonyl is bonded to C₂. However, when designated "... Rⱼ and Rᵢ are taken together to form -CH₂-CH₂-O-CO-the convention means a lactone in which the carbonyl is bonded to C₁.

The carbon atom content of variable substituents is indicated in one of two ways. The first method uses a prefix to the entire name of the variable such as "C₁-C₄", where both "1" and "4" are integers representing the minimum and maximum number of carbon atoms in the variable. The prefix is separated from the variable by a space. For example, "C₁₋₄ alkyl" represents alkyl of 1 through 4 carbon atoms, (including isomeric forms thereof unless an express indication to the contrary is given). Whenever this single prefix is given, the prefix indicates the entire carbon atom content of the variable being defined. Thus C₂-C₄ alkoxycarbonyl describes a group CH₃-(CH₂)ₙ-0-CO- where n is zero, one or 2. By the second method the carbon atom content of only each portion of the definition is indicated separately by enclosing the "Cᵢ-Cⱼ" designation in parentheses and placing it immediately (no intervening space) before the portion of the definition being defined. By this optional convention (C₁-C₃)alkoxycarbonyl has the same meaning as C₂-C₄ alkoxycarbonyl because the "C₁-C₃" refers only to the carbon atom content of the alkoxy group. Similarly while both C₂-C₆ alkoxyalkyl and (C₁-C₃)alkoxy(C₁-C₃)alkyl define alkoxyalkyl groups containing from 2 to 6 carbon atoms, the two definitions differ since the former definition allows either the alkoxy or alkyl portion alone to contain 4 or 5 carbon atoms while the latter definition limits either of these groups to 3 carbon atoms.

### II. DEFINITIONS

All temperatures are in degrees Centigrade.

TLC refers to thin-layer chromatography.

THF refers to tetrahydrofuran.

THP refers to tetrahydropyranyl.

DMF refers to dimethylformamide.

TEA refers to triethylamine.

Alcohol refers to ethyl alcohol.

MPLC refers to medium pressure liquid chromatography.

Saline refers to an aqueous saturated sodium chloride solution.

IR refers to infrared spectroscopy.

CMR refers to C-13 magnetic resonance spectroscopy, chemical shifts are reported to ppm (δ) downfield from TMS.

NMR refers to nuclear (proton) magnetic resonance spectroscopy, chemical shifts are reported in ppm (δ) downfield from tetramethylsilane.

TMS refers to trimethylsilyl.

φ refers to phenyl (C₆H₅).

MS refers to mass spectrometry expressed as m/e or mass/charge unit. [M + H]⁺ refers to the positive ion of a parent plus a hydrogen atom. EI refers to electron impact. CI refers to chemical ionization. FAB refers to fast atom bombardment.

Pharmaceutically acceptable refers to those properties and/or substances which are acceptable to the patient from a pharmacological/toxicological point of view and to the manufacturing pharmaceutical chemist from a physical/chemical point of view regarding composition, formulation, stability, patient acceptance and bioavailability.

When solvent pairs are used, the ratios of solvents used are volume/volume (v/v).

_{∼} indicates that there are 2 possible orientations for the attached group, (1) α or β when attached to the ring and (2) cis or trans when attached to a carbon atom of a double bond.

BOC refers to t-butyloxycarbonyl, -CO-O-C(CH₃)₃.

CBZ refers to carbobenzyloxy, -CO-O-CH₂-φ.

### EXAMPLES

Without further elaboration, it is believed that one skilled in the art can, using the preceding description, practice the present invention to its fullest extent. The following detailed examples described how to prepare the various compounds and/or perform the various processes of the invention and are to be construed as merely illustrative, and not limitations of the preceding disclosure in any way whatsover. Those skilled in the art will promptly recognize appropriate variations from the procedures both as to reactants and as to reaction conditions and techniques.

### EXAMPLE 1 N-Acetyl-5-nitroindoline (II)

A mixture of 5-nitroindoline (I, 12.012 g) in pyridine (100 ml) and acetic anhydride (50 ml) is stirred for 17 hr under argon. The mixture is then concentrated under reduced pressure to give the title compound, mp 175-177°; NMR (CDCl₃, 300 MHz) 8.28, 8.10, 8.01, 4.23, 3.31 and 2.28 δ; CMR (CDCl₃, 75.47 MHz) 23.95, 27.01, 49.11, 115.73, 119.93, 124.27, 132.4, 143.4 and 169.8 δ; IR (CHCl₃) 1680, 1600, 1480, 1470, 1390, 1340 and 1320 cm⁻¹.

### EXAMPLE 2 N-Acetyl-5-aminoindoline (III)

Palladium on carbon (10%, 1.110 g) is added to a mixture of N-acetyl-5-nitro indoline (II, EXAMPLE 1, 5.00 g) in ethyl acetate (freshly opened bottle, about 500 ml). The mixture is stirred under 1 atm of hydrogen (balloon) for 39 hr then filtered and the palladium on carbon is washed with methanol/ethyl acetate (20/80). The filtrate is concetrated under reduced pressure to give the title compound, mp 183-185°; NMR (CDCl₃, 300 MHz) 8.01, 6.53, 6.50, 3.98, 3.56, 3.04, and 2.17 δ; CMR (CDCl₃, 75.47 MHz) 23.86, 28.05, 48.71, 111.55, 113.73, 117.66, 132.6, 149.1 and 168.1 δ; IR (CHCl₃) 3000, 1640, 1600, 1490, 1410, 1330, and 1300 cm⁻¹.

### EXAMPLE 3 1-Acetyl-(N-carbobenzyloxy)-5-aminoindoline (IV)

Benzyl chloroformate (1.2 ml) is added to a solution of N-acetyl-5-aminoindoline (III, EXAMPLE 2, 1.4 g) and sodium bicarbonate (1.33 g) in acetone/water (40/60, 20 ml) at 0°. The mixture is stirred for 2.5 hr, then benzyl chloroformate (0.5 ml) is added. After stirring for 2.3 hr, the mixture is poured into chloroform (25 ml) and the organic layers are washed with aqueous sodium bisulfate (10%, 2x) and then washed with aqueous sodium carbonate (10%, 2x). Chloroform (about 200 ml) is added to the aqueous layers and then the organic layers are washed again with aqueous sodium bisulfate (10%), aqueous sodium carbonate (10%), then dried over magnesium sulfate, and concentrated under reduced pressure to give the title compound, 180-182°; NMR (CDCl₃, 300 MHz) 8.03, 7.38, 7.30-7.23, 6.98, 6.90, 5.09, 3.90, 3.05 and 2.09 δ ; CMR (CDCl₃, 75.47 MHz) 23.98, 28.07, 48.90, 66.89, 115.9, 117.05, 118.1, 128.25, 128.59, 132.22, 133.78, 136.21, 139.4, 154.2 and 163.39 δ; IR (CHCl₃) 3440, 1730, 1660, 1600, 1490 and 1400 cm⁻¹.

### EXAMPLE 4 1-Acetyl-(N-allyl-N-carbobenzyloxy)-5-aminoindoline (V)

Sodium hydride/mineral oil (50% w/w, 425 mg) is added to a mixture of 1-acetyl-(N-carbobenzyloxy)-5-aminoindoline (IV, EXAMPLE 3, 2.00 g) in THF (freshly distilled 80 ml). Allyl bromide (0.725 ml) is added and the mixture is refluxed for 26.5 hr under nitrogen. At the end of this time it is poured into water and extracted with ethyl acetate (3x). The organic layers are combined and dried over magnesium sulfate and concentrated under reduced pressure to a solid which is purified on a 40.63µ silica column eluting with a gradient from 100% hexane to 100% ethyl acetate. The appropriate fractions are pooled and concentrated to give the title compound, mp 108-110°; NMR (CDCl₃, 300 MHZ) 8.17, 7.29, 7.03, 5.9, 5.14, 5.1, 4.24, 4.00, 3.13 and 2.17 δ; CMR (CDCl₃, 75.47 MHz) 23.80, 27.54, 48.69, 52.23, 66.98, 116,57, 117.01, 123.13, 126.2, 127.38, 127.60, 128.13, 131.61, 133,37, 136.35, 137.3, 141.6, 155.12 and 168.33; IR (CHCl₃) 1700, 1650, 1490 and 1400 cm⁻¹; MS (m/e) 350, 215, 173 and 91; exact mass calc'd for C₂₁H₂₂N₂O₃ = 350.1630, found 350.1607.

### EXAMPLE 5 (±)-3-(5′-1-Acetylindolinyl)-5-(iodomethyl)oxazolidin-2-one (VI)

Iodine (1.94 g) is added to a mixture of 1-acetyl-(N-allyl-N-carbobenzyloxy)-5-aminoindoline (V, EXAMPLE 4, 1.3 g) in chloroform (20 ml). After stirring for 3 hr under nitrogen the mixture is poured into additional chloroform and washed with aqueous sodium thiosulfate (10%, 2x), dried over sodium sulfate and concentrated under reduced pressure to give the title compound, mp 188-190°; NMR (CDCl₃, 300 MHz) 8.17, 7.66, 7.01, 4.7, 4.15, 4.06, 3.76, 3.46, 3.36 and 2.22 δ; CMR (CDCl₃, 75.47 MHz) 6.13, 23.98, 28.00, 48.82, 51.30, 71.09, 115.621, 116.73, 117.00, 132,38, 133.7, 139.9, 154.4 and 168.44 δ; IR (CHCl₃) 1760, 1660, 1490 and 1400 cm⁻¹; MS (m/e) 386, 344, 299, 258, 216, 189, 173, 158, 145, 132; exact mass calcd for C₁₄H₁₅IN₂O₃ = 386.0129, found 386.0130.

### EXAMPLE 6 (+)-3-(5′-1-Acetylindolinyl)-5-(azidomethyl)oxazolidin-2-one (VII)

Sodium azide (1.005 g) in water (10 ml) is added to a mixture of (±)-3-(5′-1-acetylindolinyl)-5-(iodomethyl)oxazolidin-2-one (VI, EXAMPLE 5, 0.798 g) in acetone (150 ml). The mixture is refluxed under nitrogen for 42.5 hr then poured into water (225 ml). The aqueous layer is extracted with ethyl acetate (3x, 400 ml). The combined organic layers are washed with water (500 ml), with saline (300 ml) then dried over magnesium sulfate and concentrated under reduced pressure to give the title compoound, mp 165-166°; NMR (CDCl₃, 300 MHz) 8.08, 7.57, 6.9, 4.7, 4.0, 3.75, 3.62, 3.5, 3.11 and 2.14 δ; CMR (CDCl₃, 75.47 MHz) 23.92, 27.94, 47.69, 48.78, 52.97, 70.52, 115.48, 116.62, 116.82, 132.37, 133.48, 139.45, 153.97 and 168.43δ; IR (CHCl₃) 2105, 1750, 1650, 1480 and 1390 cm⁻¹; MS (m/e) 301, 273, 229, 160, 146, 132 and 117; exact mass calcd for C₁₄H₁₅N₅O₃ = 301.1174, found 301.1194.

### EXAMPLE 7 (±)-3-(5′-1-Acetylindolinyl)-5-(aminomethyl)oxazolidin-2-one (VIII)

Palladium on carbon (10%, 110 mg) is added to a mixture of (+)-3-(5′-1-acetylindolinyl)-5-(azidomethyl)oxazolidin-2-one (VII, EXAMPLE 6, 550 mg) in methanol/ethyl acetate (8/92, 130 ml). The mixture is stirred for 24 hr under 1 atm (balloon) of hydrogen. The solution is filtered and the filtrate is concentrated under reduced pressure to give the title compound, 164-165°; NMR (CDCl₃, 300 MHz) 8.08, 7.61, 6.9, 4.58, 3.98, 3.75, 3.11, 3.02, 2.90, 2.14 and 1.33; CMR (CDCl₃, 75.47 MHz) 23.96, 28.00, 44.96, 47.93, 48.82, 73.79, 115.40, 115.67, 132.29, 133.97, 139.6, 155.2 and 168.40 δ; IR (CHCl₃) 1750, 1650, 1490, 1400 and 900 cm⁻¹; MS (m/e) 275, 233, 189, 160, 147 and 117.

### EXAMPLE 8 (±)-3-(5′-1-Acetylindolinyl)-5-(acetamidomethyl)oxazolidin-2-one (IX)

A mixture of (±)-3(5′-1-acetylindolinyl)-5-(aminomethyl)oxazolidin-2-one (VIII, EXAMPLE 7, 200mg) in pyridine (5 ml) and acetic anhydride (2.5 ml) is stirred overnight. The mixture then is concentrated under reduced pressure to give a solid. The solid is recrystallized by dissolving in as little chloroform and methanol as possible then added to an equal volume of ethyl acetate and concentrated by evaporation under a nitrogen stream to give the title compound, mp 234-235°; NMR (CDCl₃, 300 MHz) 8.16, 7.58, 7.03, 6.37, 4.76, 4.04, 3.76, 3.65, 3.20, 2.23 and 2.03 δ; CMR (CDCl₃, 75.47 MHz) 23.00, 23.95, 27.99, 30.81, 41.90, 47.88, 48.81, 71.70, 115.44, 116.82, 117.13, 132.27, 133.55, 139.53, 154.45, 168.46 and 170.92 δ; IR (CHCl₃) 1755, 1670, 1490 and 1400 cm⁻¹; MS (m/e) 317, 273, 189, 172, 159, 147 and 117.

### EXAMPLE 9 1-Carbo-t-butyloxy-5-nitroindoline (II)

Di-tert-butyldicarbonate (13.4 g) is added all at once to a solution of 5-nitroindoline (I, 5.00 g) in freshly distilled THF (85 ml). The mixture is refluxed for three days then di-tert-butyldicarbonate (9.90 g) is added and the mixture refluxed overnight. The mixture is poured into water (225 ml), this is extracted with ethyl acetate (4x, total 450 ml). The combined organic layers are washed with aqueous sodium bicarbonate (5%, 500 ml), saline, dried over magnesium sulfate and concentrated under reduced pressure. The mixture of solid and oil is triturated in hexane and filtered to give the title compound, NMR (CDCl₃, 300 MHz) 8.10, 7.99, 7.85, 4.09, 3.17 and 1.58 δ; CMR (CDCl₃, 75.47 MHz) 26.38, 28.11, 48.32, 81.8, 113. 58, 120.28, 124.53, 142.38 and 151.82; IR (CHCl₃) 1710, 1605, 1490, 1395 and 1320 cm⁻¹; MS (m/e) 264, 208, 164 and 57; exact mass calcd for C₁₃H₁₆N₂O₄ = 264.1110, found 264.1117.

### EXAMPLE 10 1-Carbo-t-butyloxy-5-aminoindoline (III)

Palladium on carbon (10%, 1.0 g) is added to a mixture of 1-carbo-t-butyloxy-5-nitroindoline (II, EXAMPLE 9, 4.554 g) in ethyl acetate (freshly opened bottle, 500 ml) at 0°. The mixture is stirred under 1 atm of hydrogen (balloon) at 20-25° for 3.5 hr. The mixture is then filtered and concentrated under reduced pressure. The concentrated filtrate is taken up in ethyl acetate and washed with saline (3x). The combined aqueous layers are extracted with ethyl acetate (3x) All organic phases are combined and washed with saline, dried over magnesium sulfate and concentrated under reduced pressure to give the title compound, NMR (CDCl₃, 300 MHz) 7.64, 7.26, 6.50, 3.93, 3.48, 3.00 and 1.54 δ; CMR (CDCl₃, 75.47 MHz) 27.47, 28.42, 47.41, 77.39, 79.6, 80.6, 112.14, 113.67, 115.15, 132.4, 133.0, 134.5, 135.2, 141.71, and 152.36; IR (CHCl₃) 3360, 3440, 1680, 1485 and 1390 cm⁻¹; MS (m/e) 234, 178, 134 and 57.

### EXAMPLE 11 1-Carbo-t-butyloxy-(N-carbobenzyloxy)-5-aminoindoline (IV)

Benzyl chloroformate (2.1 ml) is added to a mixture of 1-carbo-t-butyloxy-5-aminoindoline (III, EXAMPLE 10, 3.147 g) and sodium bicarbonate (2.40 g) in acetone/water (55/45, 40 ml) at 0°. After stirring for one hour, chloroform (50 ml) is added to the mixture. The mixture is then poured into ethyl acetate (50 ml) and washed with saline. The aqueous layer is then extracted with ethyl acetate (2x) for total of 200 ml). The organic layers are combined and washed with aqueous sodium bisulfate (10%, 2 x 100 ml), aqueous sodium carbonate (10%, 2 x 100 ml), saline (100 ml), dried over magnesium sulfate then concentrated under reduced pressure to give the title compound, NMR (CDCl₃, 300 MHz) 7.74, 7.33, 7.00, 5.14, 3.92, 2.98, and 1.54 δ; CMR (CDCl₃, 300 MHz) 27.0, 28.303, 46.1, 47.52, 66.66, 73.3, 80.1, 81.0, 114.51, 118.00, 128,05, 128.38, 132.2, 132.36, 136.09, 138.3, 138.9, 152.37 and 153.60 δ; IR (CHCl₃) 3430, 1730, 1685, 1485, 1385 cm⁻¹.

### EXAMPLE 12 1-Carbo-t-butyloxy-(N-allyl-N-carbobenzyloxy)-5-aminoindoline (V)

Sodium hydride/mineral oil (50% w/w, 800 mg) is added to a mixture of 1-carbo-t-butyloxy-(N-carbobenzyloxy)-5-aminoindoline (IV, EXAMPLE 11, 4.480 g) in freshly distilled THF (180 ml). Allyl bromide (1.32 ml) is added and the mixture is refluxed for 5.5 hr under nitrogen, then it is poured into water and extracted with ethyl acetate (3x). The combined organic layers are washed with saline and dried over magnesium sulfate and then concentrated under reduced pressure to an oil which is passed over a silica column (40-63µ) eluting with a hexane - ethyl acetate gradient (100% to 100%). The appropriate fractions are pooled to give the title comound, NMR (CDCl₃, 300 MHz) 7.80, 7.29, 6.98, 5.87, 5.14, 5.10, 4.23, 3.96, 3.04 and 1.55 δ; CMR (CDCl₃, 75.47 MHz) 27.11, 28.41, 47.80, 53.54, 67.16, 77.35, 80.5, 114.55, 117.23, 126.5, 127.60, 127.80, 128.35, 132.0, 133.69, 136.4, 136.72, 141.6, 152.43 and 155.47 δ; IR (CHCl₃) 1690, 1490, 1395 and 1160 cm⁻¹.

### EXAMPLE 13 (±)-3-(5′-1-Carbo-t-butyloxyindolinyl)-5-(iodomethyl)oxazolidin-2-one (VI)

Iodine (5.512 g) is added to a mixture of 1-carbo-t-butyloxy-(N-allyl-N-carbobenzyloxy)-5-aminoindoline (V, EXAMPLE 12, 4.190 g) in chloroform (65 ml). After stirring for 3 hr the mixture is poured into chloroform (40 ml), washed with aqueous sodium thiosulfate (10%, 3 x 100 ml), dried over magnesium sulfate and concentrated under reduced pressure to a residue. The residue is passed over a silica column (40-63 µ) eluting with ethyl acetate/hexane (10/90) then eluted with chloroform. The appropriate fractions are pooled and concentrated to a solid which is recrystallized from acetone/water to give the title compound, mp 174-175°; NMR (CDCl₃, 300 MHz) 7.80, 7.53, 7.07, 4.69, 4.13, 3.98, 3.74, 3.45, 3.35, 3.09 and 1.56 δ; CMR (CDCl₃, 75.47 MHz) 6.18, 27.0, 28.26, 47.54, 51.34, 70.94, 80.6, 114.35, 115.96, 117.36, 132.17, 139.8, 152.24 and 154.01 δ; IR (CHCl₃) 1760, 1690, 1490, 1390, 1370, 1145, cm⁻¹.

### EXAMPLE 14 (±)-3-(5′-1-Carbo-t-butyloxyindolinyl)-5-(azidomethyl)oxazolidin-2-one (VII)

Following the general procedure of EXAMPLE 6 and making non-critical variations but starting with (±)-3-(5′-1-carbo-t-butyloxyindolinyl)-5-(iodomethyl)oxazolidin-2-one (VI, EXAMPLE 13), the title compound is obtained, mp 168-170°.

### EXAMPLE 15 (±)-3-(5′-1-Carbo-t-butyloxyindolinyl)-5-(aminomethyl)oxazolidin-2-one (VIII)

Following the general procedure of EXAMPLE 7 and making non-critical variations but starting with (±)-3-(5′-1-carbo-t-butyloxyindolinyl)-5-(azidomethyl)oxazolidin-2-one (VII, EXAMPLE 14), the title compound is obtained, mp 166-168.

### EXAMPLE 16 (±)-3-(5′-1-Carbo-t-butyloxyindolinyl)-5-(acetamidomethyl)oxazolidin-2-one (IX)

Following the general procedure of EXAMPLE 8 and making non-critical variations but starting with (±)-3-(5′-1-Carbo-t-butyloxyindolinyl)-5-(aminomethyl)oxazolidin-2-one (VIII, EXAMPLE 17), the title compound is obtained, mp 139-140°.

### EXAMPLE 17 (±)-3-(5′-Indolinyl)-5-(acetamidomethyl)oxazolidin-2-one (X)

Trifluoroacetic acid (0.250 ml) is added slowly over one minute to a mixture of (±)-3-(5′-1-carbo-t-butyloxyindolinyl)-5-(acetamidomethyl)oxazolidin-2-one (IX, EXAMPLE 16, 0.038 mg) in methylene chloride (3 ml). The mixture is stirred for 1.5 hr under nitrogen then poured into saturated aqueous sodium bicarbonate (30 ml). The aqueous mixture is extracted with methylene chloride (3 x for a total of 40 ml). The combined organic extracts are washed with saturated aqueous sodium bicarbonate (10 ml) and the aqueous extracts combined. The combined aqueous extracts are extracted again with methylene chloride (5 x for a total of 50 ml). The combined organic extracts are dried over magnesium sulfate and concentrated under reduced pressure to give the title compound, MS (m/e) 275, 231, 172, 159 and 147; exact mass calcd for C₁₄H₁₇N₃O₃ = 275.1270, found 275.1282.

### EXAMPLE 18 (±)-3-(5′-1-Isobutyloxyindolinyl]-5-(acetamidomethyl)oxazolidin-2-one (XI)

(±)-3-(5'-Indolinyl)-5-(acetamidomethyl)oxazolidin-2-one (X, EXAMPLE 17, 53 mg) is dissolved in methylene chloride (1.0 ml). Triethylamine (80 µl) is added. Isobutyrl chloride (25 µl) is added slowly over 30 seconds at 0°. After stirring for two hr, the mixture is added to saline (10 ml) and extracted with methylene chloride (6 x for 20 ml total). The combined organic extracts are dried over magnesium sulfate and concentrated to provide a solid. The solid is purified by passing thru a silica cartridge, eluting with chloroform (1 ml) ethyl acetate (4 ml), methanol/ethyl acetate (10/90, 27 ml). The appropriate fractions are pooled and concentrated to give the title compound, mp 200-202°.

### EXAMPLES 19-24

Following the general procedure of EXAMPLE 18 and making non-critical variations but using the appropriate R₅ group the compounds of EXAMPLES 19-24 are obtained:

| EXAMPLE | Compound Obtained |
|---|---|
| 19 | (±)-3-(5'-1-Propanoylindolinyl)-5-(acetamidomethyl)oxazolidin-2-one (XI), |
| 20 | (±)-3-(5'-1-Cyclopentylcarbonylindolinyl)-5-(acetamidomethyl)oxazolidin-2-one (XI), |
| 21 | (±)-3-(5'-1-Formylindolinyl)-5-(acetamidomethyl)oxazolidin-2-one (XI), |
| 22 | (±)-3-(5'-1-Chloroacetylindolinyl)-5-(acetamidomethyl)oxazolidin-2-one (XI), |
| 23 | (±)-3-(5'-1-Dichloroacetylindolinyl)-5-(acetamidomethyl)oxazolidin-2-one (XI) and |
| 24 | (±)-3-(5'-1-Phenylacetylindolinyl)-5-(acetamidomethyl)oxazolidin-2-one (XI) |

### EXAMPLE 25 (±)-3-(5'-1-Allylindolinyl)-5-(N-acetamidomethyl)oxazolindin-2-one (XI)

Following the general procedure of Example 18 and making non-critical variations, but using allyl bromide, the title compound is obtained.

### EXAMPLE 26 3-(5'-1-Acetylindolinyl)-5β-(aminomethyl)oxazolidin-2-one (VIII)

(±)-3-(5'-1-Acetylindolinyl)-5-(aminomethyl)oxazolidin-2-one (VIII, Example 7) is stirred with (+) or (-) tartaric acid in methylene chloride and then permitted to stand while the product crystallises out. The crystalline product is obtained by filtration and treated with triethylamine or sodium bicarbonate to obtain the free amine which is obtained by extraction with methylene chloride. The methylene chloride extract is concentrated to give the title compound.

### EXAMPLE 27 3-(5'-1-Carbo-t-butyloxyindolinyl)-5β-(aminomethyl)oxazolidin-2-one (VIII)

Following the general procedure of Example 26 and making non-critical variations but starting with the racemic mixture of Example 15, the title compound is obtained.

### EXAMPLE 28 3-(5'-1-Acetylindolinyl)-5β-(acetamidomethyl)oxazolidin-2-one (IX)

Following the general procedure of Example 8 and making non-critical variations but starting with 3-(5'-1-acetylindolinyl)-5β-(aminomethyl)oxazolidin-2-one (VIII, Example 27), the title compound is obtained.

### EXAMPLE 29 3-(5'-1-Carbo-t-butyloxyindolinyl)-5β-(aminomethyl)oxazolidin-2-one (IX)

Following the general procedure of Example 16 and making non-critical variations but starting with 3-(5'-1-carbo-t-butyloxyindolinyl)-5β-(aminomethyl)oxazolidin-2-one (VIII, Example 28), the title compound is obtained.

### EXAMPLE 30 3-(5'-Indolinyl)-5β-(acetamidomethyl)oxazolidin-2-one (X)

Following the general procedure of Example 17 and making non-critical variations but starting with 3-(5'-1-carbo-t-butyloxyindolinyl)-5β-(acetamidomethyl)oxazolidin-2-one (IX, Example 29), the title compound is obtained.

### EXAMPLE 31 3-(5'-1-Isobutyrylindolinyl)-5β-(acetamidomethyl)oxazolidin-2-one (XI)

Following the general procedure of Example 18 and making non-critical variations but starting with 3-(5'-indolinyl)-5β-(acetamidomethyl)oxazolidin-2-one (X, Example 30), the title compound is obtained.

### EXAMPLE 32 3-(5'-1-Allylindolinyl)-5β-(acetamidomethyl)oxazolidin-2-one (XI)

Following the general procedure of Example 18 and making non-critical variations but starting with 3-(5'-indolinyl)-5β-(acetamidomethyl)oxazolidin-2-one (X, Example 30) and using allyl bromide, the title compound is obtained.

### EXAMPLES 33-38

Following the general procedure of EXAMPLES 19-24 and making non-critical variations but starting with 3-(5'-indolinyl)-5β-(acetamidomethyl)oxazolidin-2-one (X, EXAMPLE 30), the compounds of EXAMPLES 33-38 are obtained:
33 3-(5'-1-Propanoylindolinyl)-5β-(acetamidomethyl)oxazolidin-2-one (XI),
34 3-(5'-1-Cyclopentylcarbonylindolinyl)-5β-(acetamido methyl)oxazolidin-2-one (XI),
35 3-(5'-1-Formylindolinyl)-5β-(acetamidomethyl)oxazolidin-2-one (XI),
36 3-(5'-1-Chloroacetylindolinyl)-5β-(acetamidomethyl)oxazolidin-2-one (XI),
37 3-(5'-1-Dichloroacetylindolinyl)-5β-(acetamidomethyl)oxazolidin-2-one (XI) and
38 3-(5'-1-Phenylacetylindolinyl)-5β-(acetamidomethyl)oxazolidin-2-one (XI)

### EXAMPLES 34-48

Following the general procedure of
1. EXAMPLES 1-7 for production of the protected aminomethyl (VIII),
2. EXAMPLES 16-18 for production of the optically active (XI),
3. For the cases with hydroxyacetyl and propyl, in addition, follow the procedures of EXAMPLES 2 or 10 (reduction of nitro to amino is the same conditions as for reduction of allyl to propyl or cleavage of a benzyl group) and making non-critical variations but starting with appropriately substituted nitroindoline (I), the compounds of EXAMPLES 39-48 are obtained:
   39 (±)-3-(5'-1-Benzoylindolinyl)-5β-(acetamidomethyl)oxazolidin-2-one (XI), mp 215-216°;
   40 (±)-3-(5'-1-Methylsulfonylindolinyl)-5β-(acetamidomethyl)oxazolidin-2-one (XI), mp 177-178°;
   41 (±)-3-(5'-1-Methylindolinyl)-5β-(acetamidomethyl)oxazolidin-2-one (XI), NMR (methanol-d₄) 7.36, 7.08, 6.49, 4.70, 4.02, 3.71, 3.51, 3.25, 2.89, 2.71 and 1.96 δ;
   42 (±)-3-(5'-1-Hydroxyacetylindoliny1)-5β-(acetamidomethyl)oxazolidin-2-one (XI), mp 207-209°;
   43 (±)-3-(5'-1-Benzyloxyacetylindolinyl)-5β-(acetamidomethyl)oxazolidin-2-one (XI), mp 181-183°;
   44 (±)-3-(5'-1-p-Chlorobenzoylindolinyl)-5β-(acetamidomethyl)oxazolidin-2-one (XI), mp 225-227°;
   45 (±)-3-(5'-1-Allylindolinyl)-5β-(acetamidomethyl)oxazolidin-2-one (XI), mp 152-153°;
   46 (±)-3-(5'-1-Propylindolinyl)-5β-(acetamidomethyl)oxazolidin-2-one (XI), NMR (CDGl₃, 300 MHz) 7.21, 7.17, 7.00, 6.37, 4.70, 3.99, 3.71, 3.56, 3.33, 2.95, 1.99, 1.60, and 0.97 δ; CMR (CDCl₃, 75.47 MHz): 11.56, 20.37, 22.83, 28.41, 41.86, 48.80, 51.07, 53.02, 71.80, 106.29, 117.48, 119.47, 127.80, 130.85, 150.34, 155.33, and 171.259 δ; IR (mineral oil mull) 3418, 1732, 1661, 1550, 1504, 1473, 1228, and 1084 cm⁻¹; MS (m/e): 317, 288, 244, 185, 173, 159, and 130; exact mass calculated for C₁₇H₂₃N₃O₃ - 317.1739, found 317.1736.
   47 (±)-3-(5'-1-Methoxyacetylindolinyl)-5β-(acetamidomethyl)oxazolidin-2-one (XI), mp 209-210°;
   48 (±)-3-(5'-1-Hexanoylindolinyl)-5β-(acetamidomethyl)oxazolidin-2-one (XI), mp 194-195°.

### EXAMPLE 49 (±)-3-(5'-1-(O-Acetyl(hydroxyacetyl)indolinyl))-5-(acetamidomethyl)-oxazolidin-2-one (XI)

The free hydroxy group of (±)-3-(5'-1-hydroxyacetylindolinyl)-5-(acetamidomethyl)oxazolidin-2-one (XI, EXAMPLE 42) is acylated as is known to those skilled in the art, NMR (CDCl₃, 300 MHz) 8.10, 7.58, 7.01, 6.49, 4.77, 4.01, 3.76, 3.65, 3.23, 2.23 and 2.04 δ.

### EXAMPLE 50 3-(5'-1-(O-Acetyl(hydroxyacetyl)indolinyl))-5β-(acetamidomethyl)oxazolidin-2-one (XI)

Following the general procedure of EXAMPLE 49 and making non-critical variations but starting with 3-(5'-1-hydroxyacetyolindolinyl)-5β-(acetamidonethyl)oxazolidin-2-one (XI, EXAMPLE 56) the title compound is obtained.

### EXAMPLE 51 (±)-3-[5'-1-(2-thienylcarbonyl)indolinyl]-5-(acetamidomethyl)oxazolidin-2-one (XI)

Following the general procedure of EXAMPLE 18 and making non-critical variations but using 2-thienylcarbonyl chloride, the title compound is obtained, mp 201-203°.

### EXAMPLE 52 3-[5'-1-(2-Thienylcarbonyl)indolinyl]-5β-(acetamidomethyl)oxazolidin-2-one (XI)

Following the general procedure of EXAMPLE 18 and making non-critical variations but using 3-(5'-indolinyl)-5β-(acetamidomethyl)oxazolidin-2-one (X, EXAMPLE 30) and 2-thienylcarbonyl chloride, the title compound is obtained.

### EXAMPLES 53-62

Following the general procedure of EXAMPLES 39-48 and making non-critical variations but using the process of EXAMPLE 26 for the resolution of the optically impure mixture of (VIII) and thereafter using the optically active (VIII), the title compounds are obtained:
53 3-(5'-1-Benzoylindolinyl)-5β-(acetamidoaethyl)oxazolidin2-one (XI),
54 3-(5'-1-Methylsulfonylindolinyl)-5β-(acetamidomethyl)oxazolidin-2-one (XI),
55 3-(5'-1-Methylindolinyl)-5β-(acetamidomethyl)oxazolidin-2-one (XI),
56 3-(5'-1-Hydroxyacetylindolinyl)-5β-(acetamidomethyl)oxazolidin-2-one (XI),
57 3-(5'-1-Benzyloxyacetylindolinyl)-5β-(acetamidomethyl)oxazolidin-2-one (XI),
58 3-(5'-1-p-Chlorobenzoylindolinyl)-5β-(acetamidomethyl)oxazolidin-2-one (XI),
59 3-(5'-1-Allylindolinyl)-5β-(acetamidomethyl)oxazolidin-2-one (XI),
60 3-(5'-1-Propylindolinyl)-5β-(acetamidomethyl)oxazolidin-2-one (XI),
61 3-(5'-1-Methoxyacetylindolinyl)-5β-(acetamidomethyl)oxazolidin-2-one (XI),
62 3-(5'-1-Hexanoylindolinyl)-5β-(acetamidomethyl)oxazolidin-2-one (XI).

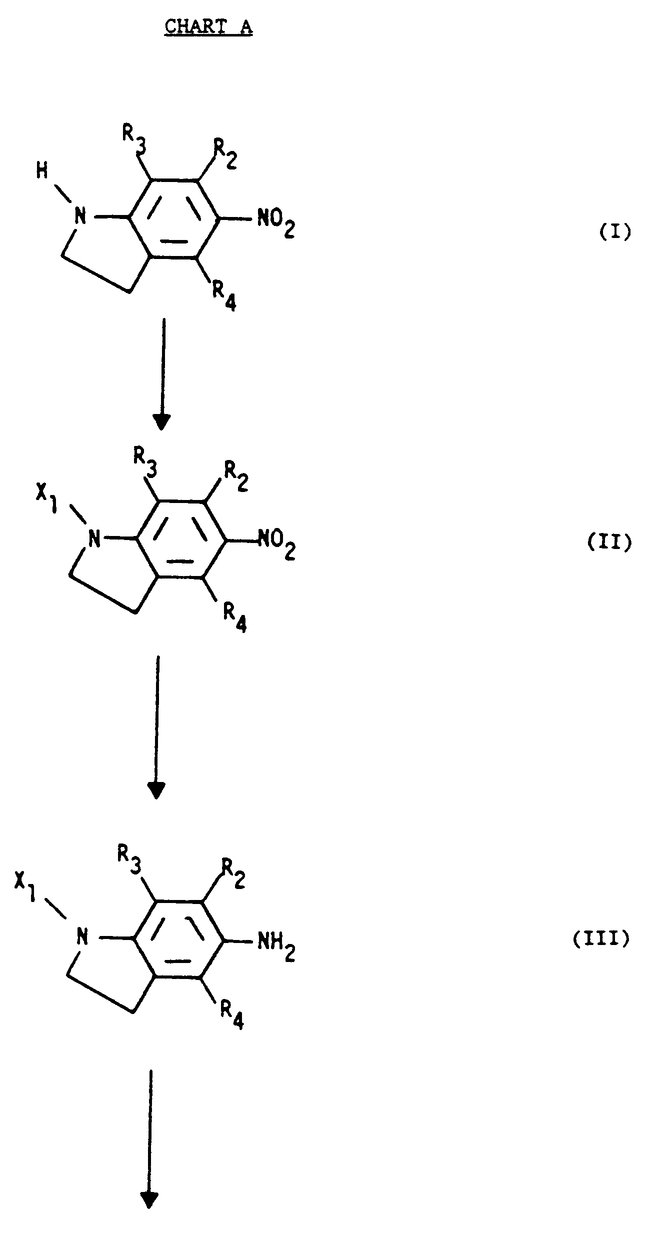

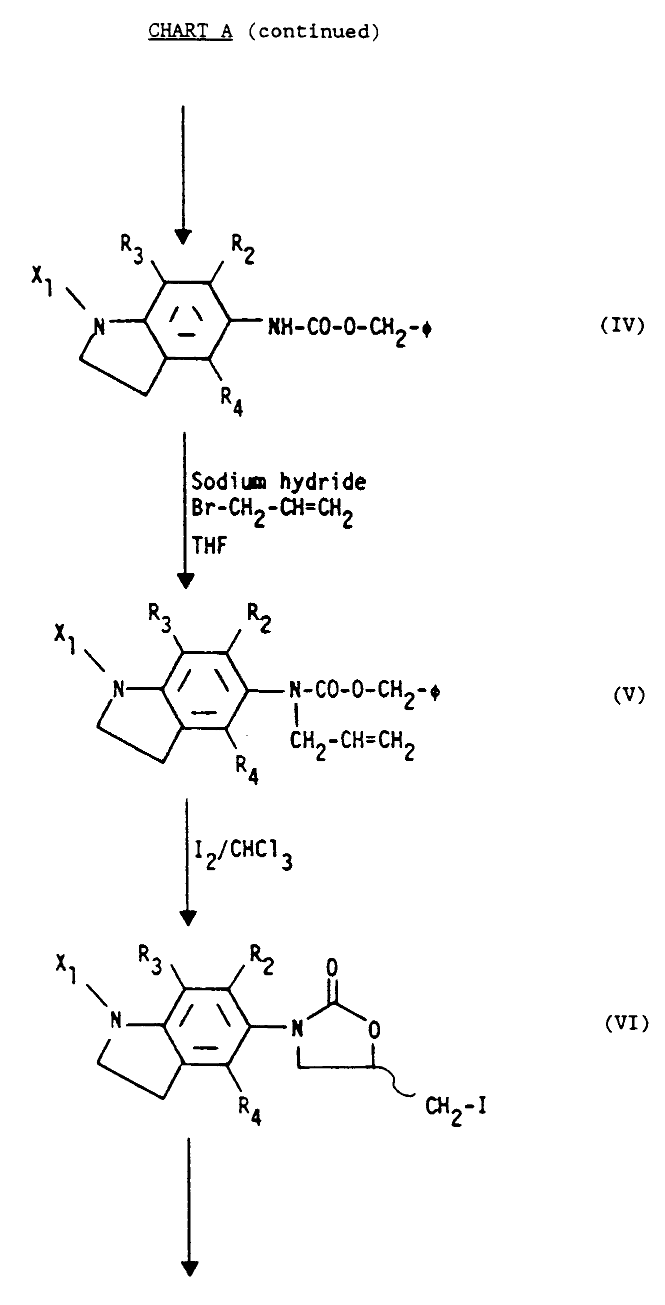

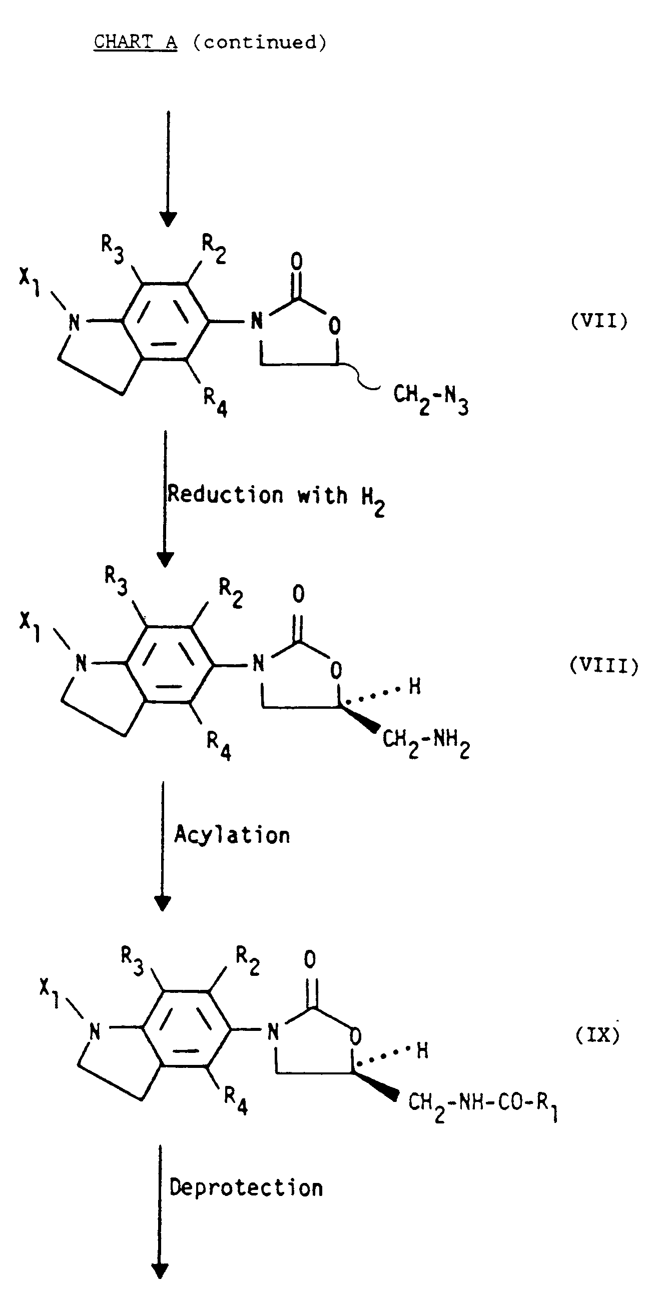

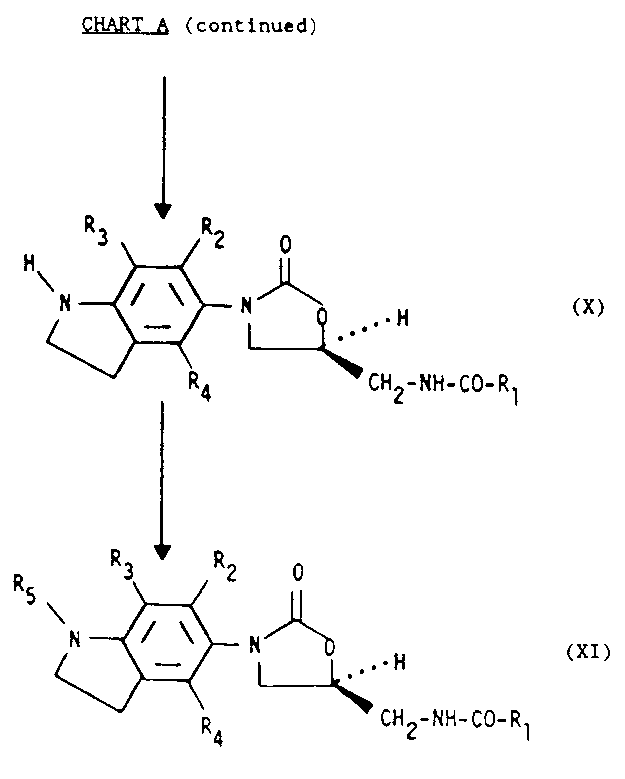

## Claims

1. A 5′-indolinyloxazolidin-2-one of formula (XI) 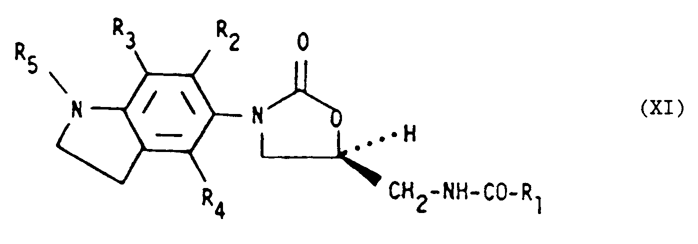 where
(I) R₁ is -H,
C₁-C₁₂ alkyl optionally substituted with 1-3 Cl,
C₃-C₁₂ cycloalkyl,
C₅-C₁₂ alkenyl containing one double bond,
- φ optionally substituted with 1-3 -OH, -OCH₃, -OC₂H₅,
-NO₂, -F, Cl, -Br, -COOH and -SO₃H, -N(R₁₋₄)(R₁₋₅) where R₁₋₄ and R₁₋₅ are the same or different and are -H, C₁-C₂ alkyl,
furanyl,
tetrahydrofuranyl,
2-thiophene,
pyrrolidinyl,
pyridinyl,
-O-R₁₋₆ where R₁₋₆ is C₁-C₄ alkyl,
-NH₂,
-NHR₁₋₆ where R₁₋₆ is as defined above,
-NR₁₋₆R₁₋₇ where R₁₋₇ is C₁-C₃ alkyl and R₁₋₆ is as defined above, and where R₁₋₆ and R₁₋₇ can be taken together with the attached nitrogen atom to form a saturated mono-nitrogen C₅-C₇ heterocyclic ring including -O- (morpholine),
-CH₂-OH,
-CH₂-OR₁₋₈ where R₁₋₈ is C₁-C₄ alkyl or -CO-R₁₋₉ where R₁₋₉ is C₁-C₄ alkyl or -φ;
(II) two of R₂, R₃ and R₄ are -H and the other of R₂, R₃ and R₄ is -H,
-F, -Cl, Br, -I,
C₁-C₆ alkyl,
-OH,
-CO-O-R₂₋₁ where R₂₋₁ is C₁-C₄ alkyl or φ,
-O-R₂₋₁ where R₂₋₁ is as defined above,
-COOH,
-COO⁻,
-CHO,
-CO-NR₂₋₂R₂₋₃ where R₂₋₂ and R₂₋₃ are the same or different and are -H, C₁-C₃ alkyl, or R₂₋₂ and R₂₋₃ are taken together with the attached nitrogen atom to form a saturated mono-nitrogen C₃-C₆ heterocyclic ring optionally containing -O-,
-C≡N,
-C≡CH,
-N≡C,
-CHOH-CH₃,
-CO-CH₃,
-SH,
-SO-CH₃,
-SO-φ,
-S-CH₃,
-S-φ,
-SO₂-CH₃,
-SO₂-φ,
-SO₃H,
-SO₂-NH₂,
-N₃,
-NO₂,
-NR_{2-4R2-5} where R₂₋₄ and R₂₋₅ are the same or different and are -H and C₁-C₃ alkyl,
-NH-CO-R₂₋₆ where R₂₋₆ is C₁-C₄ alkyl or -φ,
-NH-CO-NH₂,
-CH=CH₂,
-CH₂-CH=CH₂,
-CH=N-OH,
-CH=N-OCH₃, 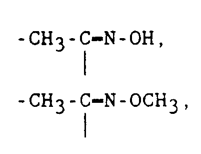 -C*H-CH₂-O* where the atoms marked with the asterisk (*) are bonded to each other resulting in the formation of a ring, where
(III) R₅ is -H,
C₁-C₁₂ alkyl,
-CH₂-φ,
-CH₂CH₂-φ,
C₃-C₇ cycloalkyl,
C₂-C₁₂ alkenyl containing from 1 thru 3 double bonds,
C₂-C₁₂ alkynyl containing 1 triple bond,
-CHO,
-CO-R₅₋₁ where R₅₋₁ is
(A) C₁-C₆ alkyl optionally substituted with 1 -O-CH₃, -φ, -COOH, -NH₂, -SO₃H or 1-3 -Cl,
(B) C₃-C₇ cycloalkyl,
(C) 2-pyridinyl, 2-thiophene, 2-thiophenemethyl or 2-pyrrole,
(D) -φ optionally substituted with 1-3
-F, -Cl, Br, -I,
C₁-C₆ alkyl,
-OH,
-CO-O-R₅₋₂ where R₅₋₂ is C₁-C₄ alkyl or -φ,
-O-R₅₋₂ where R₅₋₂ is as defined above,
-COOH,
-CO-NR₅₋₃R₅₋₄ where R₅₋₃ and R₅₋₄ are the same or different and are -H, C₁-C₃ alkyl, or R₅₋₃ and R₅₋₄ are taken together with the attached nitrogen atom to form a saturated mononitrogen C₃-C₆ heterocyclic ring optionally containing -O-,
-C≡N,
-CHOH-CH₃,
-CO-CH₃,
-SH,
-SO-CH₃,
-SO-φ,
-S-CH₃,
-S-φ,
-SO₂-CH₃,
-SO₂-φ,
-SO₃H,
-SO₂-NH₂,
-N₃,
-NO₂,
-NR_{5-5R5-6} where R₅₋₅ and R₅₋₆ are the same or different and are -H and C₁-C₃ alkyl,
-NH-CO-R₅₋₇ where R₅₋₇ is C₁-C₄ alkyl or -φ,
-CO-O-R₅₋₈ where R₅₋₈ is C₁-C₄ alkyl or -φ either optionally substituted with 1 or 2
-F, -Cl, Br, -I,
C₁-C₆ alkyl,
-OH,
-CO-O-R₅₋₂ where R₅₋₂ is as defined above,
-O-R₅₋₂ where R₅₋₂ is as defined above,
-COOH,
-CO-NR₅₋₃R₅₋₄ where R₅₋₃ and R₅₋₄ are as defined above,
-C≡N,
-CHOH-CH₃,
-CO-CH₃,
-SH,
-SO-CH₃,
-SO-φ,
-S-CH₃,
-S-φ,
-SO₂-CH₃,
-SO₂-φ,
-SO₃H,
-SO₂-NH₂,
-N₃,
-NO₂,
-NR_{5-5R5-6} where R₅₋₅ and R₅₋₆ are as defined above,
-NH-CO-R₅₋₇ where R₅₋₇ is as defined above,
-CO-N(R₅₋₉)₂ where R₅₋₉ is -H or R₅₋₈ as defined above and where the R₅₋₉'s can be taken together with the attached nitrogen atom to form a saturated mononitrogen C₃-C₆ heterocyclic ring optionally containing -O- or -NH-,
-CO-CH₂-CN,
-CO-CH₂-OH,
-CO-CH₂-O-φ where φ is optionally substituted with 1-3
-O-CH₃, 1 -NO₂ and 1 -NH₂,
-CO-CH₂-O-R₅₋₁₀ where R₅₋₁₀ is
C₁-C₆ alkyl optionally substituted with -φ,
-φ optionally substituted with 1-3 -O-CH₃, 1 -NO₂ and -NH₂,
-CO-R₅₋₁₁ where R₅₋₁₁ is C₁-C₆ alkyl, -φ optionally substituted with 1-4 -F, 1-3 -Cl, 1 -OCH₃, -OH, -NH₂, -NO₂, -CO-CH₃, -SO₂-CH₃, and -SO₂-OH,
-CO-CH(NH-CO-R₅₋₁₂)-R₅₋₁₃ where R₅₋₁₃ is -H or -CH₃ and R₅₋₁₂ is C₁-C₆ alkyl, -φ optionally substituted with 1 or 2 -OH, -OCH₃, -NO₂, -NH₂, -Cl, -F, -NH-CH₃ and -N(CH₃)₂,
-CO-CHNH₂-R₅₋₁₄ where R₅₋₁₄ is -CH(CH₃)₂, -CH₂-CH(CH₃)₂, -CHCH₃-CH₂-CH₃, -CH₂-OH, -CH(OH)-CH₃, -CH₂-φ, -CH₂-φ-OH, -CH₂-SH, -CH₂-CH₂-S-CH₃, and -CH₂-COOH,
-SO₂-CH₃,
-SO₂-φ,
-SO₃H,
and R₆ is -H and C₁-C₃ alkyl and pharmaceutically acceptable salts thereof.

2. A 5′-indolinyloxazolidin-2-one (XI) according to claim 1 where R₁ is selected from the group consisting of -H, C₁-C₆ alkyl, C₃-C₆ cycloalkyl, -OCH₃ and -CHCl₂ and where R₅ is selected from the group consisting of -CH₃, -CH₂-CH=CH₂, CH₂-C≡CH, -CHO, -CO-R₅₋₁ where R₅₋₁ is -CH₃, -C₂H₅, -CH(CH₃)₂, -CHCl₂, -CH₂-OH, -CH₂-O-CH₃, 2-thiophene and cyclopentyl.

3. A 5′-indolinyloxazolidin-2-one (XI) according to claim 1 which is
3-(5′-1-acetylindolinyl)-5β-(acetamidomethyl)oxazolidin-2-one,
3-(5′-indolinyl)-5β-(acetamidomethyl)oxazolidin-2-one,
3-(5′-1-isobutyrlindolinyl)-5β-(acetamidomethyl)oxazolidin-2-one,
3-(5′-1-propanoylindolinyl)-5β-(acetamidomethyl)oxazolidin-2-one,
3-(5′-1-cyclopentylcarbonylindolinyl)-5β-(acetamidmethyl)oxazolidin-2-one,
3-(5′-1-formylindolinyl)-5β-(acetamidomethyl)oxazolidin-2-one,
3-(5′-1-chloroacetylindolinyl)-5β-(acetamidomethyl)oxazolidin-2-one,
3-(5′-1-dichloroacetylindolinyl)-5β-(acetamidomethyl)oxazolidin-2-one,
3-(5′-1-phenylacetylindolinyl)-5β-(acetamidomethyl)oxazolidin-2-one,
3-(5′-1-(O-acetyl(hydroxyacetyl)indolinyl)-5β-(acetamidomethyl)oxazolidin-2-one,
3-[5′-1-(2-thienylcarbonyl)indolinyl)-5β-(acetamidomethyl)oxazolidin-2-one,
3-(5′-1-benzoylindolinyl)-5β-(acetamidomethyl)oxazolidin-2-one,
3-(5′-1-methylsulfonylindolinyl)-5β-(acetamidomethyl)oxazolidin-2-one,
3-(5′-1-methylindolinyl)-5β-(acetamidomethyl)oxazolidin-2-one,
3-(5′-1-hydroxyacetylindolinyl)-5β-(acetamidomethyl)oxazolidin-2-one,
3-(5′-1-benzyloxyacetylindolinyl)-5β-(acetamidomethyl)oxazolidin-2-one,
3-(5′-1-p-chlorobenzoylindolinyl)-5β-(acetamidomethyl)oxazolidin-2-one,
3-(5′-1-allylindolinyl)-5β-(acetamidomethyl)oxazolidin-2-one,
3-(5′-1-propylindolinyl)-5β-(acetamidomethyl)oxazolidin-2-one (XI),
3-(5′-1-methoxyacetylindolinyl)-5β-(acetamidomethyl)oxazolidin-2-one and
3-(5′-1-hexanoylindolinyl)-5β-(acetamidomethyl)oxazolidin-2-one.

4. A compound selected from the group consisting of a 5′-indolinyloxazolidin-2-one of formula (XIII) 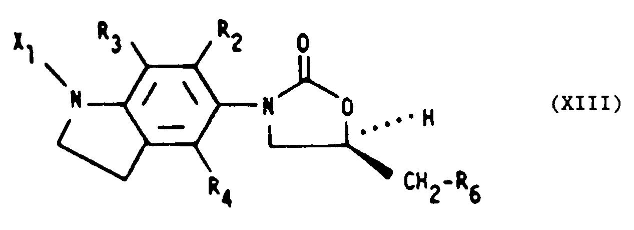 wherein R₂, R₃ and R₄ are as defined in claim 1;
X₁ is -CO-CH₃, -CO-O-C(CH₃)₃, -CO-O-CH₂-φ or -CO-O-(CH₂)₂-Si(CH₃)₃; and
R₆ is I, N₃ or NH₂;
or a salt thereof.

5. A compound according to claim 4, where X₁ is t-butyloxycarbonyl.

6. A compound according to claim 4, selected from
(±)-3-(5'-1-acetylindolinyl)-5-(iodomethyl)oxazolidin-2-one,
(±)-3-(5'-1-acetylindolinyl)-5-(azidomethyl)oxazolidin-2-one, and
3-(5'-1-acetylindolinyl)-5β-(aminomethyl)oxazolidin-2-one.

## Patentansprüche

1. 5'-Indolinyloxazolidin-2-on der Formel (XI) 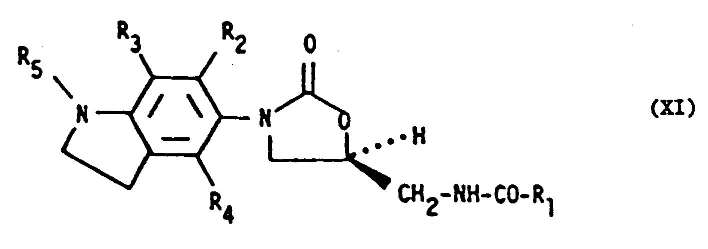 worin bedeuten:
(I) R₁ -H,
C₁-C₁₂ Alkyl, gegebenenfalls substituiert durch 1-3 Cl,
C₃-C₁₂ Cycloalkyl,
C₅-C₁₂ Alkenyl mit einer Doppelbindung,
-φ, gegebenenfalls substituiert durch 1-3 -OH, -OCH₃, -OC₂H₅, -NO₂, -F, -Cl, -Br, -COOH und -SO₃H, -N(R₁₋₄)(R₁₋₅), worin R₁₋₄ und R₁₋₅ gleich oder verschieden sein können und für -H, C₁-C₂ Alkyl stehen,
Furanyl,
Tetrahydrofuranyl,
2-Thiophen,
Pyrrolidinyl,
Pyridinyl,
-O-R₁₋₆ mit R₁₋₆ gleich C₁-C₄ Alkyl,
-NH₂,
-NHR₁₋₆ mit R₁₋₆ in der zuvor angegebenen Bedeutung,
-NR₁₋₆R₁₋₇ mit R₁₋₇ gleich C₁-C₃ Alkyl und R₁₋₆ in der zuvor angegebenen Bedeutung, wobei R₁₋₆ und R₁₋₇ zusammen mit dem Stickstoffatom, an dem sie hängen, einen gesättigten Mono-Stickstoff C₅-C₇ heterocyclischen Ring einschließlich -O- (Morpholin) bilden können,
-CH₂OH,
-CH₂-OR₁₋₈ mit R₁₋₈ gleich C₁-C₄ Alkyl oder -CO-R₁₋₉ mit R₁₋₉ gleich C₁-C₄ Alkyl oder -φ;
(II) zwei der Reste R₂, R₃ und R₄ -H und der verbleibende Rest von R₂, R₃ und R₄ -H,
-F, -Cl, Br, -I,
C₁-C₆ Alkyl,
-OH,
-CO-O-R₂₋₁ mit R₂₋₁ gleich C₁-C₄ Alkyl oder -φ,
-O-R₂₋₁ mit R₂₋₁ in der zuvor angegebenen Bedeutung,
-COOH,
-COO⁻,
-CHO,
-CO-NR₂₋₂R₂₋₃, worin R₂₋₂ und R₂₋₃ gleich oder verschieden sind und für -H, C₁-C₃ Alkyl stehen oder R₂₋₂ und R₂₋₃ zusammen mit dem Stickstoffatom, an dem sie hängen, einen gesättigten, gegebenenfalls -O-enthaltenden, Mono-Stickstoff C₃-C₆ heterocyclischen Ring bilden,
-C≡N,
-C≡CH,
-N≡C,
-CHOH-CH₃,
-CO-CH₃,
-SH,
-SO-CH₃,
-SO-φ,
-S-CH₃,
-S-φ,
-SO₂-CH₃,
-SO₂-φ,
-SO₃H,
SO₂-NH₂,
-N₃,
-NO₂,
-NR₂₋₄R₂₋₅ mit R₂₋₄ und R₂₋₅, die gleich oder verschieden sein können, gleich -H oder C₁-C₃ Alkyl,
-NH-CO-R₂₋₆ mit R₂₋₆ gleich C₁-C₄ Alkyl oder -φ,
-NH-CO-NH₂,
-CH=CH₂,
-CH₂-CH=CH₂,
-CH=N-OH,
-CH=N-OCH₃, 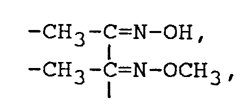 -C*H-CH₂-O*, worin die mit dem Sternchen (*) markierten Atome aneinander unter Ringbildung gebunden sind;
(III) R₅ -H,
C₁-C₁₂ Alkyl,
-CH₂-φ,
-CH₂CH₂-φ,
C₃-C₇ Cycloalkyl,
C₂-C₁₂ Alkenyl mit 1 bis 3 Doppelbindung(en),
C₂-C₁₂ Alkinyl mit einer Dreifachbindung,
-CHO,
-CO-R₅₋₁ mit R₅₋₁ gleich
(A) C₁₋₆ Alkyl, gegebenenfalls substituiert durch 1 -O-CH₃, -φ, -COOH, -NH₂, -SO₃H oder 1-3 -Cl,
(B) C₃-C₇ Cycloalkyl,
(C) 2-Pyridinyl, 2-Thiophen, 2-Thiophenmethyl oder 2-Pyrrol,
(D) -φ, gegebenenfalls substituiert mit 1-3 -F, -Cl, Br, -I,
C₁-C₆ Alkyl,
-OH,
-CO-O-R₅₋₂ mit R₅₋₂ gleich C₁-C₄ Alkyl oder -φ,
-O-R₅₋₂ mit R₅₋₂ in der zuvor angegebenen Definition,
-COOH,
-CO-NR₅₋₃R₅₋₄, worin R₅₋₃ und R₅₋₄ gleich oder verschieden sind und für -H, C₁-C₃ Alkyl stehen oder R₅₋₃ und R₅₋₄ zusammen mit dem Stickstoffatom, an dem sie hängen, einen gesättigten, gegebenenfalls -O-enthaltenden Mono-Stickstoff C₃-C₆ heterocyclischen Ring bilden,
-C≡N,
-CHOH-CH₃,
-CO-CH₃,
-SH,
-SO-CH₃,
-SO-φ,
-S-CH₃,
-S-φ,
-SO₂-CH₃,
-SO₂-φ,
-SO₃H,
-SO₂-NH₂,
-N₃,
-NO₂,
-NR₅₋₅R₅₋₆ mit R₅₋₅ und R₅₋₆, die gleich oder verschieden sein können, gleich -H oder
C₁-C₃ Alkyl,
-NH-CO-R₅₋₇ mit R₅₋₇ gleich C₁-C₄ Alkyl oder -φ,
-CO-O-R₅₋₈ mit R₅₋₈ gleich C₁-C₄ Alkyl oder -φ, beide gegebenenfalls substitutiert mit 1 oder 2
-F, -Cl, Br, -I,
C₁-C₆ Alkyl,
-OH,
-CO-O-R₅₋₂ mit R₅₋₂ in der zuvor angegebenen Bedeutung,
-O-R₅₋₂ mit R₅₋₂ in der zuvor angegebenen Bedeutung,
-COOH,
-CO-NR₅₋₃R₅₋₄ mit R₅₋₃ und R₅₋₄ in der zuvor angegebenen Bedeutung,
-C≡N,
-CHOH-CH₃,
-CO-CH₃,
-SH,
-SO-CH₃,
-SO-φ,
-S-CH₃,
-S-φ,
-SO₂-CH₃,
-SO₂-φ,
-SO₃H,
-SO₂-NH₂,
-N₃,
-NO₂,
-NR₅₋₅R₅₋₆ mit R₅₋₅ und R₅₋₆ in der zuvor angegebenen Bedeutung,
-NH-CO-R₅₋₇ mit R₅₋₇ in der zuvor angegebenen Bedeutung,
-CO-N(R₅₋₉)₂ mit R₅₋₉ gleich -H oder R₅₋₈ in der zuvor angebenen Bedeutung, wobei die beiden Reste R₅₋₉ zusammen mit dem Stickstoffatom, an dem sie hängen, einen gesättigten, gegebenenfalls -O- oder -NH- enthaltenden Mono-Stickstoff C₃-C₆ heterocyclischen Ring bilden können,
-CO-CH₂-CN,
-CO-CH₂-OH,
-CO-CH₂-O-φ, wobei φ gegebenenfalls durch 1-3 -O-CH₃, 1 -NO₂ und 1 -NH₂ substituiert ist,
-CO-CH₂-O-R₅₋₁₀ mit R₅₋₁₀ gleich C₁-C₆ Alkyl, gegebenenfalls substituiert durch -φ,
-φ, gegebenenfalls substituiert durch 1-3 -O-CH₃, 1 -NO₂ und -NH₂,
-CO-R₅₋₁₁ mit R₅₋₁₁ gleich C₁-C₆ Alkyl, -φ gegebenenfalls substituiert durch 1-4 -F, 1-3 -Cl, 1 -OCH₃, -OH, -NH₂, -NO₂, -CO-CH₃, -SO₂-CH₃ und -SO₂-OH,
-CO-CH(NH-CO-R₅₋₁₂)-R₅₋₁₃ mit R₅₋₁₃ gleich -H oder -CH₃ und R₅₋₁₂ gleich C₁-C₆ Alkyl, -φ gegebenenfalls substituiert mit 1 oder 2 -OH, -OCH₃, -NO₂, -NH₂, -Cl, -F, -NH-CH₃ und -N(CH₃)₂,
-CO-CHNH₂-R₅₋₁₄ mit R₅₋₁₄ gleich -CH(CH₃)₂, -CH₂-CH(CH₃)₂, -CHCH₃-CH₂-CH₃, -CH₂-OH, -CH(OH)-CH₃, -CH₂-φ, -CH₂-φ-OH, -CH₂-SH, -CH₂-CH₂-S-CH₃ oder -CH₂-COOH,
-SO₂-CH₃,
-SO₂-φ,
-SO₃H,
und
R₆ -H oder C₁-C₃ Alkyl,
und dessen pharmazeutisch akzeptable Salze.

2. 5'-Indolinyloxazolidin-2-on (XI) nach Anspruch 1, worin R₁ aus der Gruppe -H, C₁-C₆ Alkyl, C₃-C₆ Cyclo-alkyl, -OCH₃ und -CHCl₂ und R₅ aus der Gruppe -CH₃, -CH₂-CH=CH₂, -CH₂-C≡CH, -CHO, -CO-R₅₋₁ mit R₅₋₁ gleich -CH₃, -C₂H₅, -CH(CH₃)₂, -CHCl₂, -CH₂-OH, -CH₂-O-CH₃, 2-Thiophen und Cyclopentyl ausgewählt sind.

3. 5'-Indolinyloxazolidin-2-on (XI) nach Anspruch 1, nämlich
3-(5'-1-Acetylindolinyl)-5β-(acetamidomethyl)-oxazolidin-2-on,
3-(5'-Indolinyl)-5β-(acetamidomethyl)-oxazolidin-2-on,
3-(5'-1-Isobutyrylindolinyl)-5β-(acetamidomethyl)-oxazolidin-2-on,
3-(5'-1-Propanoylindolinyl)-5β-(acetamidomethyl)-oxazolidin-2-on,
3-(5'-1-Cyclopentylcarbonylindolinyl)-5β-(acetamidomethyl)-oxazolidin-2-on,
3-(5'-1-Formylindolinyl)-5β-(acetamidomethyl)-oxazolidin-2-on,
3-(5'-1-Chloracetylindolinyl)-5β-(acetamidomethyl)-oxazolidin-2-on,
3-(5'-1-Dichloracetylindolinyl)-5β-(acetamidomethyl)oxazolidin-2-on,
3-(5'-1-Phenylacetylindolinyl)-5β-(acetamidomethyl)oxazolidin-2-on,
3-(5'-1-(O-Acetyl(hydroxyacetyl)indolinyyl)-5β-(acetamidomethyl)-oxazolidin-2-on,
3-[5'-1-(2-Thienylcarbonyl)-indolinyl]-5β-(acetamidomethyl)-oxazolidin-2-on,
3-(5'-1-Benzoylindolinyl)-5β-(acetamidomethyl)-oxazolidin-2-on,
3-(5'-1-Methylsulfonylindolinyl)-5β-(acetamidomethyl)oxazolidin-2-on,
3-(5'-1-Methylindolinyl)-5β-(acetamidomethyl)-oxazolidin-2-on,
3-(5'-1-Hydroxyacetylindolinyl)-5β-(acetamidomethyl)oxazolidin-2-on,
3-(5'-1-Benzyloxyacetylindolinyl)-5β-(acetamidomethyl)oxazolidin-2-on,
3-(5'-1-p-Chlorbenzoylindolinyl)-5β-(acetamidomethyl)oxazolidin-2-on,
3-(5'-1-Allylindolinyl)-5β-(acetamidomethyl)-oxazolidin-2-on,
3-(5'-1-Propylindolinyl)-5β-(acetamidomethyl)-oxazolidin-2-on (XI),
3-(5'-1-Methoxyacetylindolinyl)-5β-(acetamidomethyl)oxazolidin-2-on und
3-(5'-1-Hexanoylindolinyl)-5β-(acetamidomethyl)-oxazolidin-2-on.

4. Verbindung, ausgewählt aus der Gruppe, bestehend aus einem 5'-Indolinyloxazolidin-2-on der Formel (XIII) 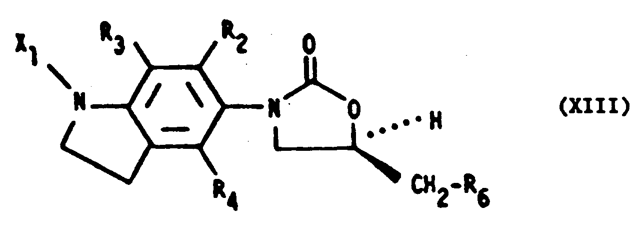 worin R₂, R₃ und R₄ die in Anspruch 1 angegebene Bedeutung besitzen,
X₁ für -CO-CH₃, -CO-O-C(CH₃)₃, -CO-O-CH₂-φ oder -CO-O-(CH₂)₂-Si(CH₃)₃ steht und
R₆ I, N₃ oder NH₂ darstellt,
oder ein Salz hiervon.

5. Verbindung nach Anspruch 4, worin X₁ für tert.-Butyloxycarbonyl steht.

6. Verbindung nach Anspruch 4, ausgewählt aus
(±)-3-(5'-1-Acetylindolinyl)-5-(iodmethyl)-oxazolidin-2-on,
(±)-3-(5'-1-Acetylindolinyl)-5-(azidomethyl)oxazolidin-2-on und
3-(5'-1-Acetylindolinyl)-5β-(aminomethyl)-oxazolidin-2-on.

## Revendications

1. 5'-indolinyloxazolidine-2-one de formule (XI) 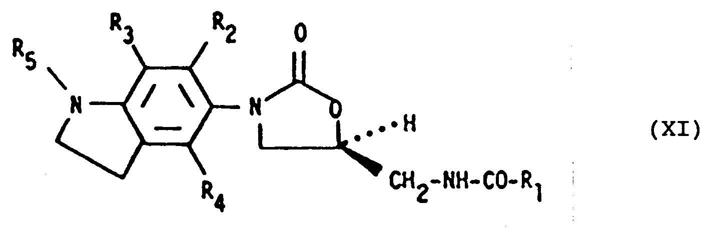 dans laquelle
(I) R₁ représente -H,
un groupe alkyle en C₁ à C₁₂ facultativement substitué avec 1 à 3 Cl,
un groupe cycloalkyle en C₃ à C₁₂,
un groupe alcényle en C₅ à C₁₂ contenant une double liaison,
un groupe -φ facultativement substitué avec un 1 à 3 groupes -OH, -OCH₃, -OC₂H₅, -NO₂, -F, -Cl, -Br, -COOH ou -SO₃H, -N(R₁₋₄) (R₁₋₅) dans lequel R₁₋₄ et R₁₋₅ sont identiques ou différents et représentent -H ou des groupes alkyle en C₁ à C₂,
un groupe furannyle,
un groupe tétrahydrofurannyle,
un groupe de thiophène,
un groupe pyrrolidinyle,
un groupe pyridinyle,
un groupe -O-R₁₋₆ dans lequel R₁₋₆ représente
un groupe alkyle en C₁ à C₄,
un groupe -NH₂,
un groupe -NHR₁₋₆ dans lequel R₁₋₆ répond à la définition précitée,
un groupe -NR₁₋₆R₁₋₇ dans lequel R₁₋₇ représente un groupe alkyle en C₁ à C₃ et R₁₋₆ répond à la définition précitée, et dans lequel R₁₋₆ et R₁₋₇ peuvent être pris conjointement avec l'atome d'azote auquel ils sont fixés pour former un noyau hétérocyclique en C₅ à C₇ monoazoté saturé comprenant un atome -O- (morpholine),
un groupe -CH₂-OH,
un groupe -CH₂-OR₁₋₈ dans lequel R₁₋₈ représente un groupe alkyle en C₁ à C₄, ou un groupe -CO-R₁₋₉ dans lequel R₁₋₉ représente un groupe alkyle en C₁ à C₄ ou un groupe -φ,
(II) deux de R₂, R₃ et R₄ représentent -H et l'autre des groupes R₂, R₃ et R₄ représente -H, -F, -Cl, Br, -I,
un groupe alkyle en C₁ à C₆,
un groupe -OH,
un groupe -CO-O-R₂₋₁ dans lequel R₂₋₁ représente un groupe alkyle en C₁ à C₄ ou -φ,
un groupe -O-R₂₋₁ dans lequel R₂₋₁ répond à la définition précitée,
un groupe -COOH,
un groupe -COO⁻,
un groupe -CHO,
un groupe -CO-NR₂₋₂R₂₋₃ dans lequel R₂₋₂ et R₂₋₃ sont identiques ou différents et représentent -H, des groupes alkyle en C₁ à C₃, ou bien R₂₋₂ et R₂₋₃ sont pris conjointement avec l'atome d'azote auquel ils sont fixés pour former un noyau hétérocyclique en C₃ à C₆ monoazoté saturé contenant facultativement un atome -O-,
un groupe -C=N,
un groupe -C=CH,
un groupe N=C,
un groupe -CHOH-CH₃,
un groupe -CO-CH₃,
un groupe SH,
un groupe -SO-CH₃,
un groupe -SO-φ,
un groupe -S-CH₃,
un groupe -S-φ,
un groupe -SO₂-CH₃,
un groupe -SO₂-φ,
un groupe -SO₃H,
un groupe -SO₂-NH₂,
un groupe N₃,
un groupe -NO₂,
un groupe NR₂₋₄R₂₋₅ dans lequel R₂₋₄ et R₂₋₅ sont identiques ou différents et représentent -H ou des groupes alkyle en C₁ à C₃,
un groupe -NH-CO-R₂₋₆ dans lequel R₂₋₆ représente un groupe alkyle en C₁ à C₄ ou -φ,
un groupe -NH-CO-NH₂,
un groupe -CH=CH₂,
un groupe -CH₂-CH=CH₂,
un groupe -CH=N-OH,
un groupe -CH=N-OCH₃,
un groupe 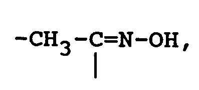 un groupe 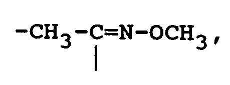 un groupe -C*H-CH₂-O* dans lequel les atomes marqués par l'astérisque (*) sont liés l'un à l'autre avec pour résultat la formation d'un noyau,
(III) R₅ représente -H,
un groupe alkyle en C₁ à C₁₂,
un groupe -CH₂-φ,
un groupe -CH₂CH₂-φ,
un groupe cycloalkyle en C₃ à C₇,
un groupe alcényle en C₂ à C₁₂ contenant 1 à 3 doubles liaisons,
un groupe alcynyle en C₂ à C₁₂ contenant une triple liaison,
un groupe -CHO,
un groupe -CO-R₅₋₁ dans lequel R₅₋₁ représente
(A) un groupe alkyle en C₁ à C₆ facultativement substitué avec un groupe -O-CH₃, -φ, -COOH, -NH₂, -SO₃-H ou 1 à 3 Cl,
(B) un groupe cycloalkyle en C₃ à C₇,
(C) un groupe 2-pyridinyle, 2-thiophène, 2-thiophèneméthyle ou 2-pyrrole,
(D) un groupe -φ facultativement substitué avec 1 à 3 groupes
-F, -Cl, Br, -I,
alkyle en C₁ à C₆,
-OH,
-CO-O-R₅₋₂ dans lequel R₅₋₂ représente un groupe alkyle en C₁ à C₄ ou -φ,
-O-R₅₋₂ dans lequel R₅₋₂ répond à la définition précitée,
-COOH,
-CO-NR₅₋₃R₅₋₄ dans lequel R₅₋₃ et R₅₋₄ sont identiques ou différents et représentent -H ou des groupes alkyle en C₁ à C₃, ou bien R₅₋₃ et R₅₋₄ sont pris conjointement avec l'atome d'azote auquel ils sont fixés pour former un noyau hétérocyclique en C₃ à C₆ monoazoté saturé contenant facultativement un atome -O-,
-C=N,
-CHOH-CH₃,
-CO-CH₃,
-SH,
-SO-CH₃,
-SO-φ,
-S-CH₃,
-S-φ,
-SO₂-CH₃,
-SO₂-φ,
-SO₃H,
-SO₂-NH₂,
-N₃,
-NO₂,
-NR₅₋₆R₅₋₆ dans lequel R₅₋₅ et R₅₋₆ sont identiques ou différents et représentent -H ou des groupes alkyle en C₁ à C₃,
-NH-CO-R₅₋₇ dans lequel R₅₋₇ représente un groupe alkyle en C₁ à C₄ ou -φ,
un groupe -CO-O-R₅₋₈ dans lequel R₅₋₈ représente un groupe alkyle en C₁ à C₄ ou -φ facultativement substitué avec un ou deux groupes
-F, -Cl, Br, -I,
alkyle en C₁ à C₆,
-OH,
-CO-O-R₅₋₂ dans lequel R₅₋₂ répond à la définition précitée,
-O-R₅₋₂ dans lequel R₅₋₂ répond à la définition précitée,
-COOH,
-CO-NR₅₋₃R₅₋₄ dans lequel R₅₋₃ et R₅₋₄ répondent aux définitions précitées,
-C=N,
-CHOH-CH₃,
-CO-CH₃,
-SH,
-SO-CH₃,
-SO-φ,
-S-CH₃,
-S-φ,
-SO₂-CH₃,
-SO₂-φ,
-SO₃H,
-SO₂-NH₂,
-N₃,
-NO₂,
-NR₅₋₆R₅₋₆ dans lequel R₅₋₅ et R₅₋₆ répondent aux définitions précitées,
-NH-CO-R₅₋₇ dans lequel R₅₋₇ répond à la définition précitée,
un groupe -CO-N(R₅₋₉)₂ dans lequel R₅₋₉ représente -H ou un groupe R₅₋₈ répondant à la définition précitée et dans lequel les groupes R₅₋₉ peuvent être pris conjointement avec l'atome d'azote auquel ils sont fixés pour former un noyau hétérocyclique en C₃ à C₆ monoazoté saturé contenant facultativement -O- ou un groupe -NH-,
un groupe -CO-CH₂-CN,
un groupe -CO-CH₂-OH,
un groupe CO-CH₂-O-φ dans lequel φ est facultativement substitué avec 1 à 3 groupes -O-CH₃, un groupe -NO₂ et un groupe -NH₂,
un groupe -CO-CH₂-O-R₅₋₁₀ dans lequel R₅₋₁₀ représente
un groupe alkyle en C₁ à C₆ facultativement substitué avec un groupe -φ,
un groupe -φ facultativement substitué avec 1 à 3 groupes -O-CH₃, un groupe -NO₂ et un groupe -NH₂,
un groupe -CO-R₅₋₁₁ dans lequel R₅₋₁₁ représente un groupe alkyle en C₁ à C₆, -φ facultativement substitué avec 1 à 4 groupes -F, 1 à 3 groupes -Cl, un groupe -OCH₃, -OH, -NH₂, -NO₂, -CO-CH₃, -SO₂-CH₃ ou -SO₂-OH,
un groupe -CO-CH(NH-CO-R₅₋₁₂)-R₅₋₁₃ dans lequel R₅₋₁₃ représente -H ou un groupe -CH₃ et R₅₋₁₂ représente un groupe alkyle en C₁ à C₆, -φ facultativement substitué avec 1 ou 2 groupes -OH, -OCH₃, -NO₂, -NH₂, -Cl, -F, -NH-CH₃ ou -N(CH₃)₂,
un groupe -CO-CHNH₂-R₅₋₁₄ dans lequel R₅₋₁₄ représente un groupe -CH(CH₃)₂, -CH₂-CH(CH₃)₂, -CHCH₃-CH₂-CH₃, -CH₂-OH, -CH(OH)-CH₃ -CH₂-φ, -CH₂-φ-OH, -CH₂-SH, -CH₂-CH₂-S-CH₃ ou -CH₂-COOH,
un groupe -SO₂-CH₃,
un groupe -SO₂-φ,
un groupe SO₃H, et R₆ représente -H ou un groupe alkyle en C₁ à C₃, et ses sels pharmaceutiquement acceptables.

2. 5'-indolynyloxazolidine-2-one (XI) suivant la revendication 1, dans laquelle R₁ est choisi entre les groupes -H, alkyle en C₁ à C₆, cycloalkyle en C₃ à C₆, -OCH₃ et -CHCl₂ et dans laquelle R₅ est choisi entre les groupes -CH₃, -CH₂-CH=CH₂, -CH₂-C=CH, -CHO, -CO-R₅₋₁ dans lequel R₅₋₁ représente un groupe -CH₃, -C₂H₅, -CH(CH₃)₂, -CHCl₂, -CH₂OH, -CH₂-O-CH₃, 2-thiophène ou cyclopentyle.

3. 5'-indolinyloxazolidine-2-one (XI) suivant la revendication 1, qui est
la 3-(5'-1-acétylindolinyl)-5β-(acétamidométhyl)oxazolidine-2-one,
la 3-(5'-indolinyl)-5β-(acétamidométhyl)oxazolidine-2-one,
la 3-(5'-1-isobutyrylindolinyl)-5β-(acétamidométhyl)oxazolidine-2-one,
la 3-(5'-1-propanoylindolinyl)-5β-(acétamidométhyl)oxazolidine-2-one,
la 3-(5'-1-cyclopentylcarbonylindolinyl)-5β-(acétamidométhyl)oxazolidine-2-one,
la 3-(5'-1-formylindolinyl)-5β-(acétamidométhyl)oxazolidine-2-one,
la 3-(5'-1-chloroacétylindolinyl)-5β-(acétamidométhyl)oxazolidine-2-one,
la 3-(5'-1-dichloroacétylindolinyl)-5β-(acétamidométhyl)oxazolidine-2-one,
la 3-(5'-1-phénylacétylindolinyl)-5β-(acétamidométhyl)oxazolidine-2-one,
la 3-(5'-1-(O-acétyl(hydroxyacétyl)indolinyl))-5β-(acétamidométhyl)oxazolidine-2-one,
la 3-[5'-1-(2-thiènylcarbonyl)indolinyl]-5β-(acétamidométhyl)oxazolidine-2-one,
la 3-(5'-1-benzoylindolinyl)-5β-(acétamidométhyl)oxazolidine-2-one,
la 3-(5'-1-méthylsulfonylindolinyl)-5β-(acétamidométhyl)oxazolidine-2-one,
la 3-(5'-1-méthylindolinyl)-5β-(acétmidométhyl)oxazolidine-2-one,
la 3-(5'-1-hydroryacétylindolinyl)-5β-(acetamidométhyl)oxazolidine-2-one,
la 3'(5'-1-benzyloryacétylindolinyl)-5β-(acétmidométhyl)oxazolidine-2-one,
la 3-(5'-1-p-chlorobenzoylindolinyl)-5β-(acétamidométhyl)oxazolidine-2-one,
la 3-(5'-1-allylindolinyl)-5β-(acétamidomethyl)oxazolidine-2-one,
la 3-(5'-1-propylindolinyl)-5β-(acetamidométhyl)oxazolidine-2-one (XI),
la 3-(5'-1-méthoxyacétylindolinyl)-5β-(acétamidométhyl)oxazolidine-2-one, et
la 3-(5'-1-hexanoylindolinyl)-5β-(acétamidométhyl)oxazolidine-2-one.

4. Composé choisi dans le groupe consistant en une 5'-indolinyloxazolidine-2-one de formule (XIII) 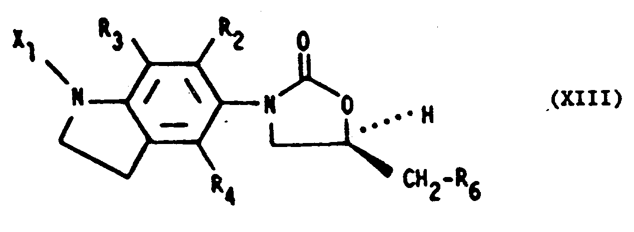 dans laquelle R₂, R₃ et R₄ répondent aux définitions suivant la revendication 1,
X₁ représente un groupe -CO-CH₃, -CO-O-C(CH₃)₃, -CO-O-CH₂-φ ou -CO-O-(CH₂)₂-Si(CH₃)₃ ; et
R₆ représente I, N₃ ou un groupe NH₂ ; et ses sels.

5. Composé suivant la revendication 4, dans lequel X₁ représente un groupe tertiobutylorycarbonyle.

6. Composé suivant la revendication 4, choisi entre
la(±)-3-(5'-1-acétylindolinyl)-5-(iodométhyl)oxazolidine-2-one,
la (±)-3-(5'-1-acétylindolinyl)-5-(azidométhyl)oxazolidine-2-one, et
la 3-(5'-1-acétylindolinyl)-5β-(aminométhyl)oxazolidine-2-one.
